# EUROPEAN PATENT APPLICATION

(11) **EP 2 382 992 A1**
(43) Date of publication of application: **02.11.2011**
(21) Application number: 10729239.3
(22) Date of filing: 08.01.2010
(51) Int. Cl.: A61K 45/00, A61K 31/713, A61K 48/00, A61P 3/04, A61P 3/10, A61P 43/00

(54) **PHARMACEUTICAL COMPOSITION FOR TREATING OBESITY OR DIABETES**

(30) Priority: 08.01.2009 JP 2009002275
(71) Applicant: Shionogi & Co., Ltd., Osaka 541-0045 (JP)
(72) Inventor: IWATA, Mana, Toyonaka-shi Osaka 561-0825 (JP); MAEKAWA, Kazuhiko, Osaka-shi Osaka 553-0002 (JP); YOSHIDA, Tetsuya, Sapporo-shi Hokkaido 001-0021 (JP)
(74) Representative: Denison, Christopher Marcus
(86) International application number: PCT/JP2010/050131
(87) International publication number: WO 2010/079819

(57) **Abstract**

The invention is intended to provide a pharmaceutical composition for treating or preventing obesity or type II diabetes and a method for treatment for them. A pharmaceutical composition comprising an Acsl-1 inhibitor of this invention as an active ingredient is useful for prevention or treatment of obesity or type II diabetes.

## Description

### Field of the Invention

This invention relates to a new use of Acsl-1. In more detail, this invention relates to a screening method with Acsl-1 to search a preventive or therapeutic agent for obesity or type II diabetes, a method for treating obesity or type II diabetes and a pharmaceutical composition for preventing or treating obesity (including the weight management for obesity) or type II diabetes.

### Background Art

Obesity is defined as the condition that adipose tissues systemically increased and occurs when the amounts of energy taken in is larger than the amounts of energy consumed over a long period of time. Obesity can be classified into visceral adiposity and subcutaneous adiposity. Visceral adiposity is obesity that the accumulation of intra-abdominal fat around greater omentum or mesentery increases. It is one of curses which cause diabetes (especially type II diabetes accompanied by insulin resistances), arteriosclerosis, liver disease, heart disease or the like and a big problem in the modern society.

Diabetes is a disease accompanied by a sustained hyperglycemic conditions and it is supposed that it occurs as a result of the action of many environmental and genetic factors. The major modulator of blood glucose in the body is insulin and hyperglycemia occurs by insulin deficiency or excess of the various-factors inhibiting the action (for example, genetics factors, lack of exercise, obesity, stress or the like). There are mainly two kinds of diabetes and it is classified into type I diabetes caused by a decrease of pancreatic insulin secretory function by an autoimmune disease or the like and type II diabetes caused by a decrease of pancreatic insulin secretory function by the pancreatic exhaustion accompanied by a sustained high insulin secretion. It is said that 95 % or more of the Japanese diabetic patients are type II diabetic. Today, an increase in the number of patients associated with the changes in lifestyle becomes a problem.

Enzymes belonging to an acyl-CoA synthetase family (hereinafter abbreviated as ACS) are enzymes that convert long-chain fatty acid to acyl CoA. Since acyl CoA serves as a substrate in intracellular lipid synthesis and fatty acid degradation or elongation reaction, ACS plays a central role in intracellular lipid metabolism and also lipid-dependent intercellular signaling. Moreover, ACS also relates to uptake of fatty acid outside of the adipose (Non-patent Document 1).

As for ACS, five isozymes whose substrate selectivity or intercellular localizations are different (ACS1, 3, 4, 5, 6) have been identified (Non-patent Document 2). Acsl-1 (GenBank: NM_001995) which is one of enzymes of this family is mainly expressed in liver or adipose tissue and catalyzes the synthesis of triglyceride (TG). Acsl-1 may be written as Acs1.

TriacsinC is known as an ACS inhibitor and it was reported that this compound inhibits 1, 3 and 4 of five isozymes (Non-patent Document 3). As for this compound, it was also reported that it inhibits TG accumulation in a human hepatoma cell line, HuH7 cells, (Non-patent Document 4) and that it inhibits diacylglycerol, cholesterol ester and phospholipid synthesis in normal human dermal fibroblasts, CCD cells (Non-patent Document 5).

It has been reported that Acsl-1 relates to various cancers (for example, Patent Documents 1 to 3). It was also reported that it can be used as a biomarker of liver cirrhosis, hepatic fibrosis (Patent Document 4) or bronchial asthma (Patent Document 5).

Furthermore, Patent Document 6 discloses antisense compounds targeted to Acsl-1. It is described in the description that these compounds are useful for treatment of diabetes or obesity. However, it is based on expectation that because Acsl-1 is an enzyme which captures fatty acid in cells, the deletion of this enzyme may possess a similar phenotype as a fatty acids transportation protein (FATP1). Moreover, referring to the described Examples, they discloses only a design method of antisense and a method for confirming the activity and it has not been confirmed what kind of a pharmacological effect can be seen by knockout or knockdown of this gene.

However, although it is common knowledge for people skilled in the art, the function of a target is not necessarily correspond with its in vivo effect and, in fact, the effect does not become clear without a pharmacological evaluation.

For example, although SCD-1 (Stearoyl-CoA Desaturase-1) is an enzyme which is next to Acsl-1 on a metabolic map and relates to lipid metabolism, it was shown that the effect of weight reduction could not confirm even in liver-specific SCD-1 knockout mice (Non-patent Document 6).

Furthermore, it was reported that when Acsl-1 was overexpressed in mouse liver, the amount of triglyceride or accumulation of fatty acid in liver increased, but there is no change in body weight, food intake, liver weight and fat weight (Non-patent Document 7). From the result that weight change is not shown by this overexpression, it can be said that the new findings are unexpected.

### Prior Art Document

### Patent Document

Patent Document 1: WO07/010628
Patent Document 2: WO07/117038
Patent Document 3: WO03/3004989
Patent Document 4: JP2007-252366
Patent Document 5: JP2004-121218
Patent Document 6: WO04/016749

### Non-patent Document

Non-patent Document 1: Biocheical.J, Vol. 323, p.1-12, 1997
Non-patent Document 2: J.Nutr, Vol. 132, p.2123-2126, 2004
Non-patent Document 3: Biochemistry, Vol. 44, p. 1635-1642, 2005
Non-patent Document 4: J.Lipid.Res, Vol. 48, p. 1280-1292, 2007
Non-patent Document 5: Biochem.J, Vol. 324, p. 529-534, 1997
Non-patent Document 6: Cell Metabolism, Vol. 6, 484-496, 2007
Non-patent Document 7: Am J Physiol Endocrinol Metab, Vol. 291, p. 737-744, 2006

### Disclosure of Invention

### Problems to be solved by the Invention

This invention is intended to provide a pharmaceutical composition for preventing or treating obesity or type II diabetes and a method for treating obesity or type II diabetes.

### Means for Solving the Problem

As a result of extensive research to solve the above problems, these inventors firstly found that increase of body weight was suppressed and glucose level was decreased by inhibiting Acsl-1 expression in liver. This invention was achieved based on these findings.

Namely, this invention relates to
(1) a pharmaceutical composition for preventing or treating obesity and/or type II diabetes characterized by comprising an Acsl-1 inhibitor as an active ingredient,
(2) the composition of the above (1), wherein the inhibitor is a functional nucleic acid which suppresses Acsl-1 expression,
(3) the composition of the above (2), wherein the functional nucleic acid is a siRNA,
(4) the composition of the above (3), wherein the siRNA has the suppressive effect on lipid droplet accumulation,
(5) the composition of the above (3), wherein the siRNA can suppress gene expression of Acsl-1 by 80% or more,
(6) the composition of the above (5), wherein the siRNA is a siRNA having a sequence selected from the group consisting of SEQ ID NO: 17 to 49 as a RNA,
(7) a method for treatment of obesity or type II diabetes characterized by administering an effective amount of an Acsl-1 inhibitor to a mammal,
(8) the method for treatment of the above (7), wherein the inhibitor is a functional nucleic acid which suppresses Acsl-1 expression,
(9) the method for treatment of the above (8), wherein the functional nucleic acid is a siRNA,
(10) a screening method for a therapeutic agent of obesity or type II diabetes by using the inhibition of expression or action of Acsl-1 as an indicator,
(11) a siRNA or derivative thereof wherein RNA has a sequence selected from the group consisting of SEQ ID NO: 17 to 49,
(12) the siRNA of the above (11), further comprising a 3'-end overhang,
(13) use of an Acsl-1 inhibitor for the manufacture of a pharmaceutical composition for preventing or treating obesity and/or type II diabetes,
(14) use of the above (13), wherein the inhibitor is a functional nucleic acid which suppresses Acsl-1 expression,
(15) use of the above (14), wherein the functional nucleic acid is a siRNA,
(16) use of the above (15), wherein the siRNA has the suppressive effect on lipid droplet accumulation,
(17) use of the above (15), wherein the siRNA can suppress gene expression of Acsl-1 by 80% or more,
   and
(18) use of the above (17), wherein the siRNA is a siRNA having a sequence selected from the group consisting of SEQ ID NO: 17 to 49 as a RNA, or derivative thereof.

### Effect of the Invention

According to this invention, a strong effect of prevention or treatment by administering an Acsl-1 inhibitor can be obtained. Therefore, a pharmaceutical composition or a method for treatment of this invention is very useful for prevention or treatment of obesity or type II diabetes.

### Brief Description of the Drawings

[Figure 1] The positions of 27 sequences selected for design of mouse Acsl-1 variant 3 ORF and shRNA were shown.
[Figure 2] The comparison result of suppressive effects on Acsl-1 expression in vitro (shRNA screening) is shown. The case which a negative control was introduced was set as 100%, and suppressive ratios on Acsl-1 expression when each shRNA was introduced were shown. Gene transfer efficiency was adjusted by measuring SEAP activity as an internal control reporter. The types of shRNA were shown on the horizontal axis.

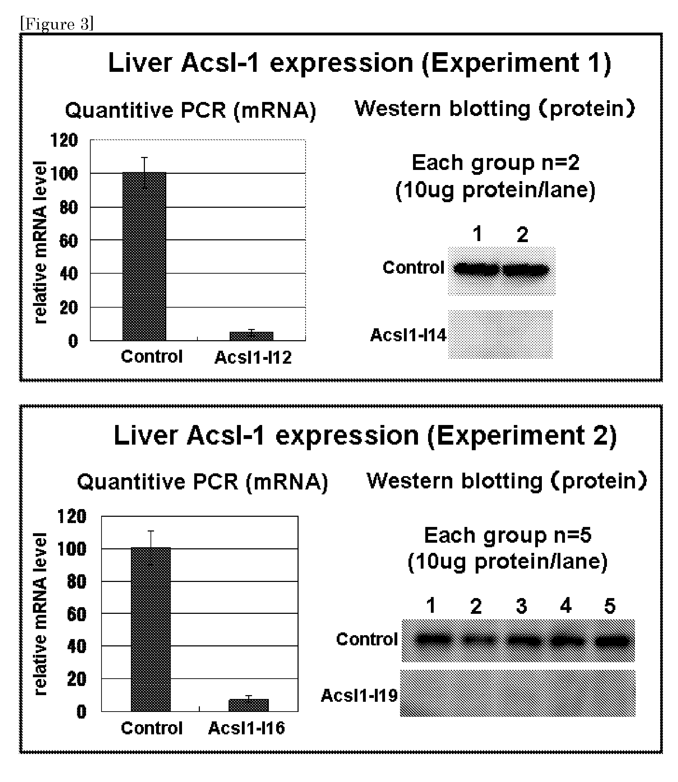

[Figure 3] The result of expression analysis of liver Acsl-1 is shown. The Acsl-1 expression in the liver of DIO mice administered HD-Ad was analyzed on the mRNA level (quantitative PCR) and the protein level (western blot). mRNA levels were adjusted by measuring GAPDH as an internal standard and the expression levels calculated when a control was set as 100% are shown. The experiment of administration of HD-Ad-U6 (Control) or HD-Ad-U6-Acsl1-I12 (Acsl1-I12) is shown as an Experiment 1 and the experiment of administration of HD-Ad-U6 (Control) or HD-Ad-U6-Acsl1-I16 (Acsl1-I16) is shown as Experiment 2 (hereinafter the same shall apply).
[Figure 4] The suppressive effects on weight gain by suppressing Acsl-1 expression were shown. The time courses of weights (left figures) and weight gain ratios (right figures) of DIO mice administered HD-Ad were shown as graphs. The vertical axis represented weights or weight gain ratios and horizontal axis represented days after virus administration. The weight gain ratios were calculated on the condition that the weight after 3 days of virus administration was set as 100%.
[Figure 5] The effect on food intake by suppressing Acsl-1 expression is shown. The time courses of food intake of DIO mice administered HD-Ad were shown as graphs. The vertical axis represented food intake and horizontal axis represented days after virus administration. Food intake means a figure which the amount of food ingested a day divided by weight of the mouse.
[Figure 6] The improvement effect on hyperglycemia by suppressing Acsl-1 expression is shown. Glucose levels in DIO mice before HD-Ad administeration and after 4 weeks of the administeration were shown as graphs.
[Figure 7] The effect on GOT by suppressing Acsl-1 expression is shown. GOT values in DIO mice before HD-Ad administeration and after 4 weeks of the administeration were shown as graphs.
[Figure 8] The improvement of insulin resistance by suppressing Acsl-1 expression is shown. Insulin levels in the blood of DIO mice before HD-Ad administeration and after 4 weeks of the administeration were shown as graphs.
[Figure 9] Lowering of total cholesterol in the blood by suppressing Acsl-1 expression is shown. Total cholesterol levels in the plasma of DIO mice before HD-Ad administeration and after 4 weeks of the administeration were shown as graphs.
[Figure 10] The improvement of fatty liver by suppressing Acsl-1 expression is shown. Mouse liver triglyceride levels in DIO mice before HD-Ad administeration and after 4 weeks of the administeration were shown as graphs.
[Figure 11] Free cholesterol levels in liver of DIO mice after 4 weeks of HD-Ad administeration were shown as graphs.
[Figure 12] Free fatty acid levels in liver of DIO mice after 4 weeks of HD-Ad administeration were shown as graphs.
[Figure 13] The consensus sequence listing in mRNA of human Acsl-1 and mRNA of mouse Acsl-1.
[Figure 14] The consensus sequence listing in mRNA of human Acsl-1 and mRNA of mouse Acsl-1.
[Figure 15] The consensus sequence listing in mRNA of human Acsl-1 and mRNA of mouse Acsl-1.
[Figure 16] The consensus sequence listing in mRNA of human Acsl-1 and mRNA of mouse Acsl-1.
[Figure 17] The consensus sequence listing in mRNA of human Acsl-1 and mRNA of mouse Acsl-1.
[Figure 18] The comparison result of suppressive effects of synthetic Acsl-1 siRNAs in HepG2 is shown. The synthesis siRNAs (#41, 53, 56 and 64,202, 207) that Acsl-1 mRNA expressions is less than the red dotted line were shown the suppressive efficiency more than Hs_ACSL1_4_HP/siRNA (Acsl-1 PC), and are considered to be expectable as anti-obesity and hyperglycemic therapeutic agent.

### Mode for Carrying Out the Invention

Hereinafter, embodiments of this invention are explained in detail.

### (A) A pharmaceutical composition and a method for treatment of this invention

One embodiment of this invention is "a pharmaceutical composition for preventing or treating obesity or type II diabetes characterized by comprising an Acsl-1 inhibitor as an active ingredient" or "a method for treatment of obesity or type II diabetes characterized by administering an effective amount of an Acsl-1 inhibitor". The "Acsl-1" is a well-known protein (GenBank: NM_001995). A DNA sequence of Acsl-1 is described in SEQ ID NO: 1 of a sequence listing and the amino acid sequence is described in SEQ ID NO: 2. "Acsl-1" in this invention is not limited to these sequences, and as long as the function of a polypeptide of SEQ ID NO: 2 is maintained, it is not limited by the number of mutations or the mutation positions of amino acid or DNA.

An "Acsl-1 inhibitor" in this invention means a substance which can inhibit expression of Acsl-1 gene (especially, a human Acsl-1 gene) or activity of the gene product. Examples of the inhibitor are a substance which affects transcription or translation of the gene, a substance which affects the activity of the gene product and the like. An Acsl-1 inhibitor also includes an inhibitor of an Acsl-1 promoter. Examples of the inhibitor are a nucleic acid such as an antisense, a siRNA or a vector comprising thereof and the like; a polypeptide such as a dominant inhibitor (dominant negative), an antibody (an antibody specifically binding to the epitope in a polypeptide comprising consecutive amino acids sequence of SEQ ID NO: 2 and the like), an enzyme and the like; a catalytic RNA such as a ribozyme and the like; an aptamer; a chemical molecule of low molecular weight (e.g., its molecular weight is less than 2000Da) and the like. These inhibitors can be selected by a screening method, for example, a method disclosed in this description.

A preferable embodiment of an "Acsl-1 inhibitor" in this invention is a "functional nucleic acid". The "functional nucleic acid" means a nucleic acid which can suppress expression of an endogenous gene such as intracellular DNA, mRNA or the like, or an innate function of DNA or mRNA by acting in a series of processes to synthesize protein, for example, a transcription or translation. For example, it is an antisense, siRNA, shRNA, miRNA, ribozyme, expression vector comprising thereof or the like. The functional nucleic acid of this invention is a nucleic acid which can suppress Acsl-1 expression. The suppression of expression means suppression of 70% and more of innate expression level of mRNA or protein, preferably 80% and more in comparison with a control.

A preferable embodiment of a "functional nucleic acid" in this invention is an antisense. In more detail, it is an antisense oligonucleotide which hybridizes to arbitrary position in a nucleotide sequence of SEQ ID NO: 1 and inhibits Acsl-1 expression. This antisense oligonucleotide hybridizes to preferably at least about 15 consecutive nucleotides in a nucleotide sequence of SEQ ID NO: 1. The above antisense oligonucleotide comprising an initiation codon in at least 15 consecutive nucleotides as above is more preferable.

The term "antisense nucleic acid" used in this description includes both a fully complementary nucleotide to a target sequence, and a nucleotide having 1 or several mismatch(es) of the nucleotide as long as the antisense nucleic acid can specifically hybridize to a target sequence. For example, an antisense nucleic acid of this invention includes a polynucleotide having at least about 70% and more, preferably at least about 80% and more, and more preferably at least about 90% and more homology over a range of at least 15 consecutive nucleotides. A well-known algorithm in this field of the invention can be used for the decision of homology. Furthermore, a derivative or modified product of an antisense oligonucleotide can be used as an antisense oligonucleotide in this invention. Examples of the modified product include a lower alkyl phosphonate modification such as a methyl-phosphonate type or ethyl-phosphonate type, a phosphorothioate modification and a phosphoroamidate modification, but it is limited to them.

Another preferable embodiment of a "functional nucleic acid" in this invention is a siRNA. A siRNA of this invention means a siRNA which suppresses a gene expression of Acsl-1. Specifically, it is a siRNA which can suppress 70% and more and preferably 80% and more of gene expression of Acsl-1 in comparison with a control. It is because it is thought that a siRNA with such inhibition ratio has the suppressive effect on variability in body weight, elevation of blood glucose level and lipid droplet accumulation. It is known that a siRNA induces a sequence specific suppression of a gene expression called RNA interference. Generally, siRNA consists of a double stranded RNA part and a 3' end overhang part of a sense strand and/or antisense strand. However, 3' end overhang part is not essential and a double stranded siRNA without an overhang part (blantend type) is also included in this invention.

The above 3' end overhang means a 3' end part projected from the both ends of 19 base pairs when two of 21-mer RNA strands that 19 mers from 5' end is a complementary sequence pair and form a double strand. The number of constitutive nucleotides is not particularly limited, but about 2 is preferable. As a sequence of 2 bases of 3' end overhang of siRNA of this invention, for example, the one whose both ends are TT (thymine • thymine) is preferable. A sequence of 2 bases of the above 3' end overhang is not limited to this, and it may be any natural nucleic acid base (adenine, guanine, thymine, cytosine or uracil), or a natural or artificial well-known modified base as long as it does not substantially affect the suppressive effect on gene expression, variability in body weight, elevation of blood glucose level, lipid droplet accumulation and the like. As a nucleotide of the above 3' end overhang part, a ribonucleotide can be usually used. But it is not limited to this, and a deoxyribonucleotide, modified ribonucleotide or another well known nucleotide analog can be used as long as it does not substantially affect the suppressive effect on gene expression, variability in body weight, elevation of blood glucose level, lipid droplet accumulation and the like. Furthermore, in a double stranded siRNA of this invention, the above 3' end overhang can be replaced with 5' end overhang as occasion demands.

A siRNA can be designed by the well known method for a person skilled in the art. For example, a strand which the selected DNA sequence (19 to 21 bases are desirable) is transformed to RNA sequence directly (sense strand) and the antisense strand are combined to make a double stranded RNA part and an overhang part is added. A siRNA of this invention is designed based on Acsl-1, and preferably DNA sequence of human Acsl-1 (SEQ ID NO: 1). It is not particularly limited as long as it is a siRNA which can suppress Acsl-1 expression. It is desirable that the suppression of expression is specific to Acsl-1. The "specific" means that the off-target effect is suppressed and preferably there is no off-target effect. That is, it is said that very high specificity which RNAi does not preferably have an effect on genes except for Acsl-1 gene is shown. It can identify whether it is specific or not by carrying out BLAST search which is open to the public.

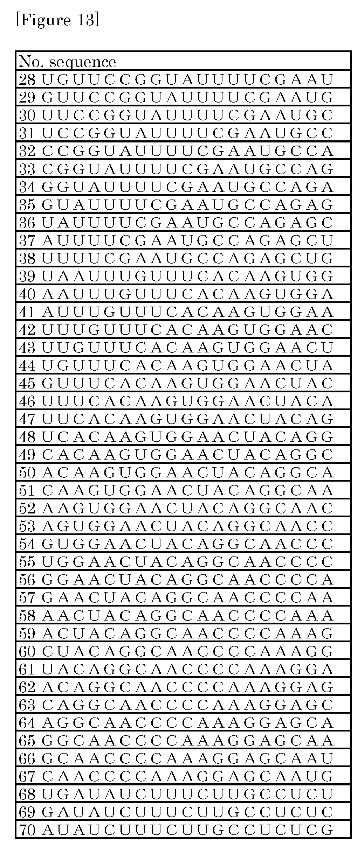

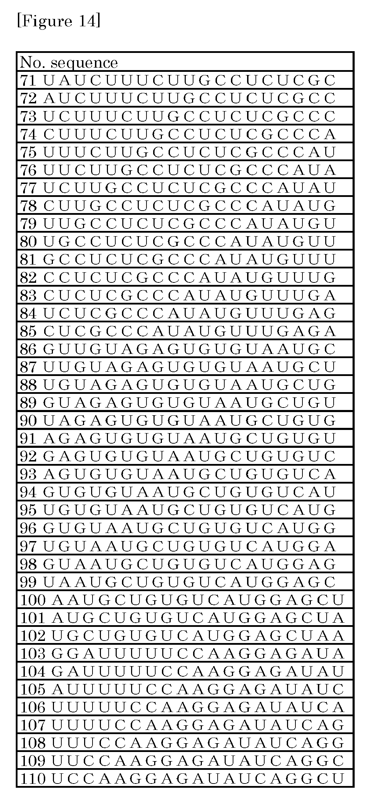

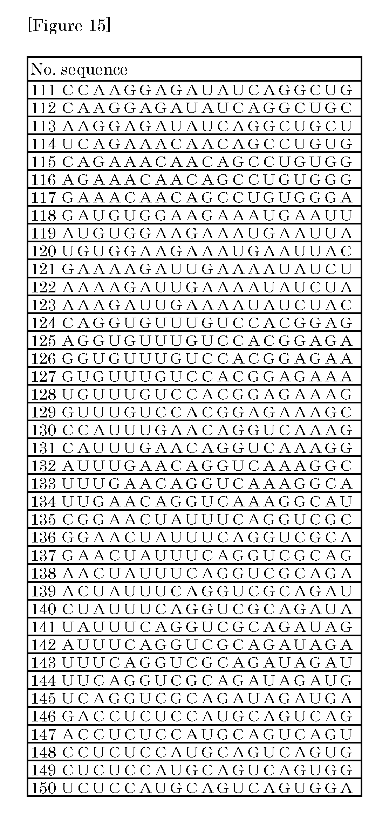

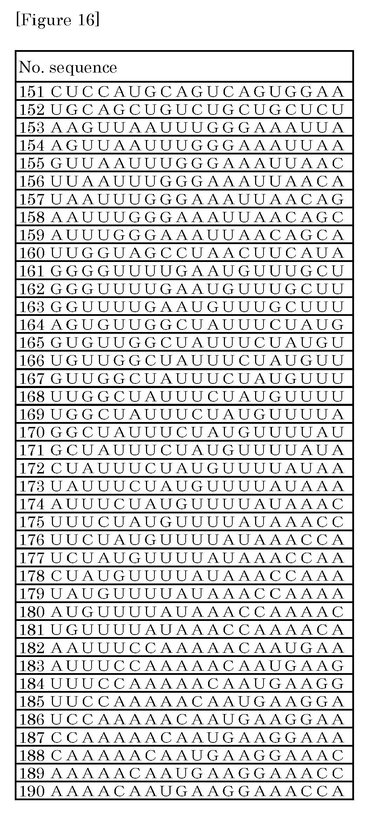

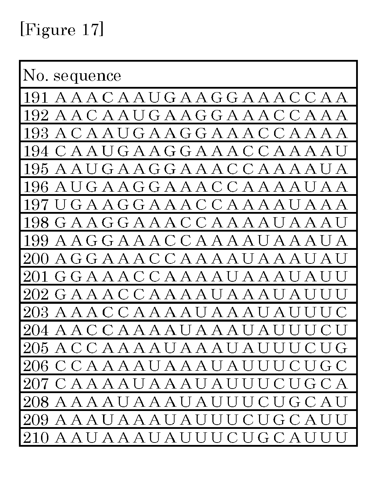

As a nucleic acid sequence comprised in a double stranded siRNA of this invention, 33 kinds of No.41, 53, 56, 64, 81, 98, 100, 101, 102, 111, 112, 117, 118, 119, 121, 125, 126, 137, 138, 139, 150, 153, 161, 163, 164, 165, 166, 178, 190, 195, 201, 202 and 207 described in the following Table 1 (or Figure 13 to 17) (respectively, a sequence of SEQ ID NO: 17 to 49 in a sequence listing) are preferably used, and No. 53, 100, 118, 119, 125, 165, 139, and 163 are more preferable.

This invention includes a "derivative" of a siRNA. The "derivative" of a siRNA means that one or several, for example, at least 2, at least 4, or at least 6, and preferably 3 and less, 4 and less, 5 and less, 6 and less, 10 and less, 15 and less, or 20 and less nucleotide substitution(s), deletion(s) and/or addition(s) exist in the above nucleic acid as long as it can suppress Acsl-1 gene specific gene expression. Furthermore, the term "derivative" includes a chemical derivative of a nucleic acid in a nucleotide base, sugar or phosphate group.

The term "RNA" in this description means a molecule comprising at least one ribonucleotide residue. "Ribonucleotide" means a nucleotide having a hydroxyl group at 2' position of β-D-ribo-furanose part. The term includes an altered RNA. As such alternation, for example, it can be included that a nonnucleotide substance is added to the end or inside of RNA, for example, 1, 2 and more nucleotide(s) of RNA. The nucleotide in RNA molecule of this invention can include a nonstandard nucleotide, for example, an unspontaneous nucleotide, chemical synthesized nucleotide, deoxynucleotide and the like.

A method for preparing a siRNA of this invention can be an in vitro chemical or enzymatic synthesis, or in vivo synthesis. Although the method is not particularly limited, it is preferable to carry out chemical synthesis by a well-known method. When a siRNA is the synthesized double stranded siRNA, its concentration regulation becomes easy and avoidance of the interferon response on the occasion of clinical application becomes easy. Moreover, there is also an advantage in safety because it is easy to prevent contamination. For example, when No.41 of 21-base overhang double stranded siRNA of this invention is produced, first, 21-mer RNA strand which 2-base overhang is added to 3' end of a sequence of SEQ ID NO: 17 in a sequence listing and 21-mer RNA strand which 2-base overhang is added to 3' end of a complementary sequence of a sequence of the above SEQ ID NO: 17 are chemical synthesized respectively. Next, the above two RNA strands are paired under the condition for pairing to obtain 21-base double stranded siRNA of this invention. It is preferable to arbitrarily purify by a well-known method when it is used, if necessary.

Additionally, a siRNA of this invention also includes any molecule having the same effect as a siRNA of this invention in an administered subject. As such molecule, for example, it is a shRNA. A shRNA means a short hairpin structured RNA and it is a RNA molecule with a stem loop structure because a part of single strand region forms complementary strand with another region. A shRNA whose double stranded RNA part has the same structure as a siRNA of this invention is also included in a siRNA of this invention. DNA which can express a siRNA of this invention by administering to a subject is also included in a siRNA of this invention. The DNA is used by constructing an expression vector (e.g., a vector such as adenovirus, adeno-associated virus, herpesvirus, lentivirus and the like) into which DNA coding a siRNA is inserted.

These inventors confirmed suppression of weight gain ratios and lowering of blood glucose level by administering a siRNA which can suppress Acsl-1 expression to mammals as an Acsl-1 inhibitor. Furthermore, they confirmed that a siRNA which can suppress Acsl-1 expression has the suppressive effect on lipid droplet accumulation. Here, suppression of lipid droplet accumulation means that excess fat accumulation can be suppressed in adipose cells, liver or skeletal muscle, and improvement of obesity or insulin resistance can be expected according to this phenomenon. These findings suggests what an Acsl-1 inhibitor is administered at an effective amount or a pharmaceutical composition comprising an Acsl-1 inhibitor as an active ingredient is useful for treatment or prevention of obesity (especially, visceral adiposity type obesity) or diabetes (especially, type II diabetes).

"Obesity" in this description means obesity which fat is accumulated in a viscus or under the skin. An inhibitor of this invention has especially a prominent effect on visceral adiposity type obesity which fat is accumulated around viscera such as greater omentum, mesentery or the like. When fat increases in a viscus or the like, TNFα (tumor necrosis factor) or free fatty acid releasing from an adipose cell reduces action of insulin in distal cells. Then, the subject becomes insulin resistance, blood sugar is elevated and type II diabetes can be induced. Moreover, in visceral adiposity type obesity, lipid in blood also increases and arteriosclerosis can be occurred with. An Acsl-1 inhibitor of this invention can be useful for prevention or treatment of obesity as well as the weight management for obesity, or diabetes (especially, type II diabetes with insulin resistance) or arteriosclerosis associated with obesity.

When an active ingredient of an Acsl-1 inhibitor is a functional nucleic acid, it is desirable that it is modified to improve the therapeutic effect, or that it is comprised in a transport carrier such as liposome or the like. To improve the effect of a functional nucleic acid as a therapeutic agent, a modified product or analog of the nucleotide can be introduced. For example, the effect is improvement of nuclease resistance and/or cellular permeability. The nuclease resistance is obtained by any well-known method in this field which does not inhibit a physiological activity of antisense, siRNA, shRNA and/or ribozyme. Examples of modifications which can be added to oligonucleotide to improve nuclease resistance are modification of phosphorus or oxygen of a hetero atom in phosphate backbone. For example, they are methyl phosphoric acid, phosphorothioate, phosphorodithioate, morpholino oligomer and the like. Moreover, stability for nuclease can be drastically enhanced while maintaining the physiological activity by another well-known modification in this field.

When an active ingredient of an Acsl-1 inhibitor is a functional nucleic acid, it is desirable that an expression vector, especially a mammal expression vector, is used for in vivo expression, especially expression in a body of a subject required for treatment of this invention. The expression vectors are well known in this field and preferably include plasmid, cosmid and virus expression system. Examples of the preferable virus expression system are adenovirus, retroviruses, lentivirus and the like. Furthermore, a method for introducing the vector in a cell or tissue is well known in this field. Preferable examples are transfection, lipofection, electroporation, infection by a recombinant virus vector and the like.

For treatment of obesity or type II diabetes, although the above Acsl-1 inhibitor can be used as it is, it is usually preferable that a pharmaceutical composition comprising the inhibitor as an active ingredient is manufactured with 1 or 2 kinds or more of pharmaceutically acceptable additives for drug formulation and administered. When the Acsl-1 inhibitor is used as a pharmaceutical composition, it can be formulated as a tablet, capsule, elixir, microcapsule or injection such as an axenic solution, suspension and freeze-dried formulation according to a standard method.

An administration method of an Acsl-1 inhibitor can be oral or parenteral administration. The embodiment of parenteral administration is not particularly limited and an intravenous administration, intramuscular administration, intraperitoneal administration, subcutaneous administration or the like can be used.

Although the dosage of an Acsl-1 inhibitor is different depending on a subject, administration method or the like, for example, for an oral administration, to a patient (60 kg), it is about 0.1 to 100 mg per day, preferably about 1.0 to 50 mg and more preferably about 1.0 to 20 mg. For a parenteral administration, for example, to a patient (60 kg), it can be about 0.01 to 30 mg per day, preferably about 0.1 to 20 mg and more preferably about 0.1 to 10 mg by intravenous injection.

### (B) A screening method of this invention

Another embodiment of this invention is a screening method for a therapeutic agent of obesity or type II diabetes by using the inhibition of expression and/or action of Acsl-1 as an indicator. As mentioned above, these inventors found that suppression of gene expression of Acsl-1 is deeply related to obesity or type II diabetes. From this, it is thought that Acsl-1 relates to progression of obesity or type II diabetes, and a substance with capacity to suppress gene expression of Acsl-1 (mRNA expression) and reduce the production of Acsl-1, or a substance with capacity to lower action of Acsl-1 is useful as an active ingredient for prevention or treatment of obesity or type II diabetes.

A screening method of this invention explained as below is to obtain an active ingredient of a therapeutic or preventive agent of obesity or type II diabetes (hereinafter, these also are collectively referred to as "an anti-obesity agent or the like") by searching (B-1) a substance with capacity to suppress gene expression of Acsl-1 (mRNA expression), (B-2) a substance with capacity to reduce the production of Acsl-1, or (B-3) a substance with capacity to lower action of Acsl-1 from test substances.

Candidate substances which can be an active ingredient of an anti-obesity agent or the like include a nucleic acid, peptide, protein, organic compound (including a low molecular compound and a high molecular compound), inorganic compound or the like. A screening method of this invention can be carried out with a sample comprising these candidate substances (these are collectively referred to as "a test substance"). Here, a sample comprising a candidate substance includes a cell extract, expression product of gene library, microbiological culture supernatant, fungus body component or the like.

### (B-1) A screening method for a substance which suppresses gene expression of Acsl-1

The screening method is a method to search a substance with capacity to suppress gene expression of Acsl-1 from test substances by using expression of Acsl-1 mRNA as an indicator, and obtain it as an active ingredient of an anti-obesity agent or the like.

Specifically, the method can be carried out with the following step (1) to (3).
(1) a step to bring a test substance into contact with a cell which can express Acsl-1 gene,
(2) a step to measure expression of Acsl-1 gene in the cell contacted with the test substance, and
(3) a step to select the test substance which the above measured quantity is smaller than expression of Acsl-1 gene in a control cell with which a test substance is not contacted.

Regardless of natural or recombinant, a cell which can express Acsl-1 gene can be used as a cell for the screening method. In addition, the origin of Acsl-1 gene is also not particularly restricted. Although the gene derived from human, mammal except for human such as mouse or another species can be used, Acsl-1 gene derived from human is preferred.

Moreover, a transformant, which is prepared to be able to express Acsl-1 mRNA by introducing an expression vector having cDNA of Acsl-1 gene according to the standard method, can be used. In addition, a cell for the screening method includes a tissue which is an aggregate of cells.

In step (1) of (B-1) of a screening method of this invention, although a condition to bring a test substance into contact with a cell which can express Acsl-1 gene is not particularly restricted, it is preferable to choose a culture condition (temperature, pH, medium composition or the like) which the cell is not be killed and Acsl-1 gene can be expressed.

A screening of a candidate substance can be carried out, for example, by bring a test substance into contact with a cell which can express Acsl-1 gene under the above condition to search a substance which suppresses expression of Acsl-1 gene to reduce the mRNA expression. Specifically, it is used as an indicator that expression of Acsl-1 mRNA when a cell which can express Acsl-1 gene is cultured under the presence of a test substance is smaller than expression of Acsl-1 mRNA (control expression) when a cell which can express Acsl-1 gene and corresponds to the above is cultured under the absence of a test substance, and the test substance contacted with the cell can be selected as an candidate substance.

The measurement of expression of Acsl-1 mRNA (detection, quantitative determination) can be carried out by measuring expression of Acsl-1 mRNA in a cell which can express Acsl-1 gene according to a well-known method such as northern blotting, RT-PCR, Real-time qPCR or the like with an oligonucleotide having a complementary sequence to a base sequence of the Acsl-1 mRNA or the like, or DNA array.

### (B-2) A screening method for a substance which lowers Acsl-1 production

The screening method is a method to search a substance with capacity to reduce Acsl-1 production from test substances by using Acsl-1 production as an indicator, and obtain it as an active ingredient of an anti-obesity agent or the like.

Specifically, the method can be carried out with the following step (1') to (3').
(1') a step to bring a test substance into contact with a cell which can produce Acsl-1 or cellular fraction prepared from this cell,
(2') a step to measure Acsl-1 production in the cell or the cellular fraction contacted with the test substance, and
(3') a step to select the test substance which the above measured quantity is smaller than Acsl-1 production in a control cell with which a test substance is not contacted (a cell which can produce Acsl-1) or a cellular fraction with which a test substance is not contacted (it prepares from a cell which can produce Acsl-1).

Regardless of natural or recombinant, a cell which can express Acsl-1 gene and produce Acsl-1 can be used as a cell for the screening method (including a control cell). In addition, the origin of Acsl-1 gene is also not particularly restricted. Although the gene derived from human, mammal except for human such as mouse or another species can be used, Acsl-1 gene derived from human is preferred.

Moreover, a transformant, which is prepared to be able to produce Acsl-1 by introducing an expression vector having cDNA of Acsl-1 gene according to the standard method, can be used. In addition, a cell for the screening method includes a tissue which is an aggregate of cells.

In step (1') of (B-2) of a screening method of this invention, although a condition to bring a test substance into contact with a cell which can produce Acsl-1 is not particularly restricted, it is preferable to choose a culture condition (temperature, pH, medium composition or the like) which the cell is not be killed, Acsl-1 gene can be expressed and Acsl-1 can be produced.

A screening of a candidate substance can be carried out, for example, by bring a test substance into contact with a cell which can produce Acsl-1 or the cellular fraction under the above condition to search a substance which reduces Acsl-1 I production. Specifically, it is used as an indicator that Acsl-1 production when a cell which can produce Acsl-1 or the cellular fraction is cultured under the presence of a test substance is smaller than Acsl-1 production (control production) when a cell which can produce Acsl-1 and corresponds to the above or the cellular fraction is cultured under the absence of a test substance, and the test substance contacted with the cell or cellular fraction can be selected as an candidate substance.

The measurement of Acsl-1 production (detection, quantitative determination) can carried out by measuring the amount of Acsl-1 obtained from a cell which can produce Acsl-1 or the cellular fraction according to a well-known method such as western blotting, immunoprecipitation method, ELISA or the like with an antibody against the Acsl-1 (anti-Acsl-1 antibody).

### (B-3) A screening method for a substance which lowers the action of Acsl-1

The screening method is a method to search a substance with capacity to lower Acsl-1 action from test substances by using Acsl-1 action as an indicator, and obtain it as an active ingredient of an anti-obesity agent or the like.

Specifically, the method can be carried out with the following step (1") to (3").
(1") a step to bring a test substance into contact with a cell which can produce Acsl-1 or cellular fraction prepared from this cell,
(2") a step to detect Acsl-1 action in the cell or the cellular fraction contacted with the test substance, and
(3") a step to select the test substance which the above detected action is lower than Acsl-1 action in a control cell (a cell which can produce Acsl-1) or a cellular fraction (it prepares from a cell which can produce Acsl-1) with which a test substance is not contacted.

As the cell for the screening and a method or condition to contact with a test substance in step (2"), what was described for a screening method of (B-2) mentioned above can be used in the same way.

A screening of a candidate substance can be carried out by bring a test substance into contact with a cell which can produce Acsl-1 or the cellular fraction under the above condition to search a substance which lowers Acsl-1 action. Specifically, it is used as an indicator that Acsl-1 action when a cell which can produce Acsl-1 or the cellular fraction is cultured under the presence of a test substance is lower than Acsl-1 action (control action) when a cell which can produce Acsl-1 and corresponds to the above or the cellular fraction is cultured under the absence of a test substance, and the test substance can be selected as an candidate substance.

Here, Acsl-1 action is, for example, acyl -CoA synthetase activity. This measurement itself can be measured according to well known methods (e.g., J.Biol.Chem, 256, 5702-5707, 1981).

A substance selected by a screening method of the above (B-1) to (B-3) has the action to reduce Acsl-1 production by suppressing expression of Acsl-1 gene in a cell, or to lower Acsl-1 action and can be used as an active ingredient of a composition to prevent obesity or type II diabetes, or the progression, or a composition to improve obesity or type II diabetes. A candidate substance selected by the above screening method can be further screened with a pathological non-human animal with obesity or type II diabetes. The selected candidate substance can be tested in drug efficacy test or safety test with a pathological non-human animal with obesity or type II diabetes, or clinical test for a patient (human) with obesity or type II diabetes or a patient (human) who is in the previous status. By conducting these tests, a more practical active ingredient of a preventive or therapeutic composition for obesity or type II diabetes can be selected and obtained.

The selected substance can be industrially produced, if necessary after structural analysis, by a chemical synthesis, biological synthesis (including fermentation) or genetic operation depending on the kind of the substance. It can be used for creation of a preventive or therapeutic composition for obesity or type II diabetes.

Although this invention is explained by the following examples, these examples are exemplified to understand this invention better, and these examples are not intended to limit the scope of this invention.

### Example 1

### Design of shRNA and preparation of double strand oligo DNA

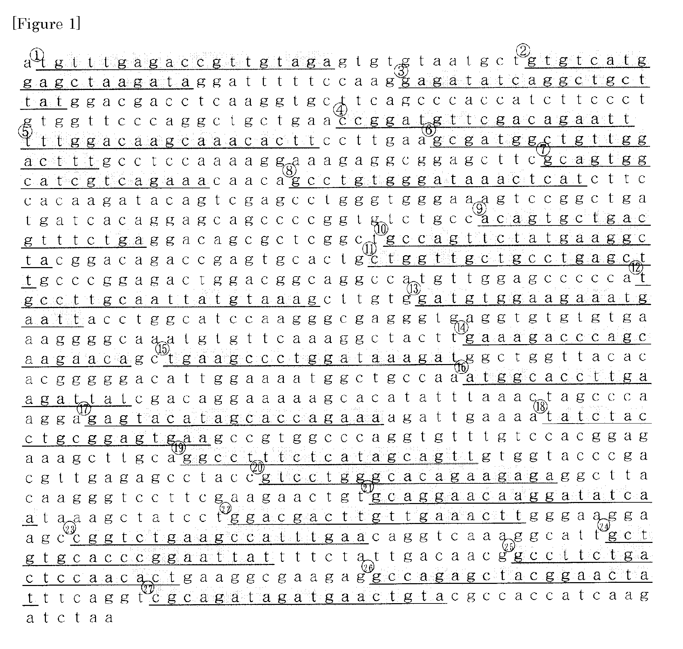

As follows, shRNA to the mouse Acsl-1 variant 3 ORF (Refseq ID XM_991228) were designed. At first, as the sense target sequence, under the condition that four bases must be thymine or adenine in seven bases of 3' end and four consecutive thymine bases were not connected. 27 sequences to which 19 bases consecutive in the above gene were completely corresponding were selected (Fig. 1). The complementary strand to these each sense target sequence was assumed to be an anti-sense target sequence. Next, top strand oligo DNA that had a loop sequence (TTCAAGAGA or ATCAAGAGA) between the sense target sequence and the anti-sense target sequence, and that had a TTTTTT sequence which is a Pol III terminator sequence in 3' end of the anti-sense target sequence was designed. In addition, a BamH I site in 5' end and a Not I site in 3' end were introduced and it was assumed a top strand oligo DNA-BN. The complementary sequence to this top strand oligo DNA-BN was assumed to be bottom strand oligo DNA-NB. The top strand oligo DNA-BN and bottom strand oligo DNA-NB were dissolved in the TE buffer (composition: 10 mM Tris-HCl, 1mM EDTA (pH 8.0)) as becoming 100µM, and then 10uL was put in each microtube, in addition, NaCl was added so that the final concentration might become 0.1M. The microtube contained this mixture was put in heat-block set to 100 degrees, heated for four minuets, and then left until returning to the room temperature, and cooling, and double strand oligo DNA was prepared.

### Example 2

### Cloning of mouse Acsl1 gene and construction of the gene expression vector

Total RNA was treated with DNase I and collected from liver of C57BL/6J (Japan Charles River) with RNeasy Mini Kit (QIAGEN) according to the appended manual. Following, cDNA was synthesized with SuperScriptIII First Strand Synthesis System (invitrogen) according to the appended manual. The Mouse Acsl-1 (variant 3) gene fragment inserted a Not I site in 5' end and a Xho I site in 3' end was obtained by PCR with the cDNA as a template DNA, Acsl-1 cloning primer (SEQ ID NO: 3 and 4) and Phusion High-Fidelity DNA polymerase (FINNZYMES) under the condition that repeated 10 times the cycle: 98 °C 10 sec, 68 °C 10 sec, and 72 °C 1 min. The expression vector was produced by the ligation of the obtained fragment and pCR3.1 (invitrogen) that were treated with Not I and Xho I.

### Example 3

### Construction of shRNA expression vector

pShuttle vector (Clontech) was digested with Xba I and Spe I to eliminate CMV promoter region and was inserted in human U6 promoter. It was assumed a pShuttle-U6. The shRNA expression vector was produced by inserting double strand oligo DNA made by a method in Example 1 into this pShuttle-U6 which was treated with BamH I and Not I.

### Example 4

### In vitro, an examination of the knockdown effect of Acsl-1 expression (shRNA screening)

HEK293 cells were co-transfected with the mouse Acsl-1 expression vector produced in Example 2 and the shRNA expression vector or pShuttle-U6 as a control produced in Example 3. And the knockdown ratio of Acsl-1 expression by shRNA sequences was compared by measuring the amount of Acsl-1 mRNA. HEK293 cells were maintained in DMEM containing 10% FCS (Fetal Calf Serum) under the condition of 5 % CO2 and 37 °C. The day before the transfection, HEK293 cells were seeded in 12 well plates. After 24 hours, they were co-transfected with 400 ng of the shRNA expression vector, 600 ng of the Acsl-1 expression vector or pShuttle-U6 and 100 ng of the secreted form of alkaline phosphatase expression vector as an internal control reporter by using FuGene (Roche) according to the appended manual. 4 days after the transfection, medium were collected and measured SEAP activity by using BD Great EscAPe™ SEAP Chemiluminescence Detection Kit (Clontech) according to the appended manual. Following, total RNA was treated with DNase I and collected from the cells with RNeasy Mini Kit (QIAGEN) according to the appended manual. cDNA was synthesized with SuperScriptIII First Strand Synthesis System (invitrogen) according to the appended manual. Using synthesized cDNA as a template, the amount of Acsl-1 mRNA was quantified by SYBR Green Real-Time PCR. Using SYBR Green PCR Master Mix (ABI) as a reaction reagent, Real-Time PCR was carried out by Applied Biosystems 7500 Real-Time PCR systems (ABI). At this time, the sequences of the used Real-Time quantitative PCR primers are shown in SEQ ID NO: 5 and 6.

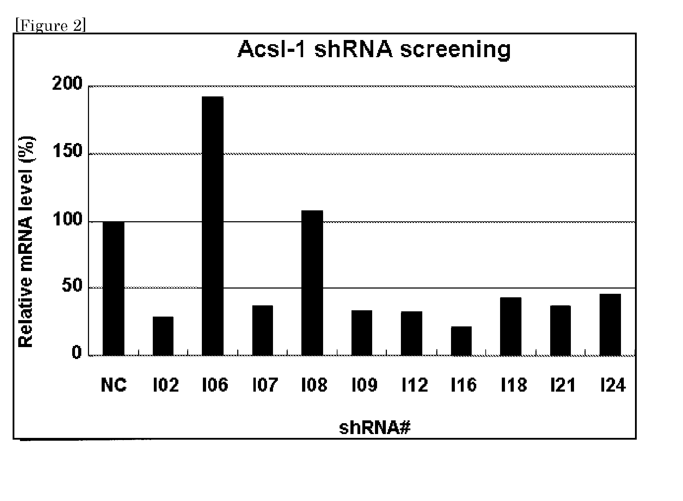

Figure 2 shows a part of the results. As the result of this examination, shRNA sequences whose knockdown ratio is great (for example, more than 50 %) were inserted into Helper dependent adenovirus vector (HD-Ad vector) and used.

### Example 5

### Construction and preparation of HD-Ad vector

pC4HSU-PISce I-ICeu I vector was produced by inserting a linker containing PI-Sce I and I-Ceu I restriction enzyme sites into pC4HSU vector (Microbix Biosystems Inc.) which was treated with Asc I. pShuttle-U6, pShuttle-U6-Acsl1-I12 made based on a base sequence shown in Figure 1; No.12, and pShuttle-U6-Acsl1-I16 made based on a base sequence shown in Figure 1; No.16 were treated with PI-Sce I and I-Ceu I and the region containing U6 promoter and shRNA sequence was cut off. pC4HSU-U6, pC4HSU-U6-Acsl1-I12, and pC4HSU-U6-Acsl1-I16 were produced by ligating the fragment cut off and pC4HSU-PISce I-ICeu I vector treated with PI-Sce I and I-Ceu I. Using these vectors, HD-Ad-vector was prepared according to the appended manual. HD-Ad-U6-Acsl1-12 is able to express siRNA containing double strand RNA that has the base sequence of UGCCUUGCAAUUAUGUAAA (SEQ ID NO: 7) in liver. On the other hand, HD-Ad-U6-Acsl1-16 is able to express siRNA containing double strand RNA that has the base sequence of AUGGCACCUUGAAGAUUAU (SEQ ID NO: 8) in liver.

### Example 6

### Experiment of mouse liver specific Acsl-1 knockdown

Diet induced obesity (DIO) mice were produced by having given high fat diet (60 % kal fat: TestDiet) to male C57BL/6J mice (Japan Charles River) at 7 weeks of age for 7 weeks and more. As data before HD-Ad were administered, DIO mice (14 weeks of age, feeding of high fat diet for 7 weeks) were measured body weight, food intake (each day), blood glucose (6 hours fasting), and collected plasma (6 hours fasting). The blood glucose level was measured with GLUCOCARD DIAmeter (arkray) according to the appended manual. 50 µL of whole blood was added to 2 µL of 0.5 M EDTA and centrifuged at 9000 rpm for 1 min. After that, the supernatant were collected as plasma samples. Following, HD-Ad 9 X 1011 vp were injected to tail vein of DIO mice which the data was measured before administration (15 weeks of age, feeding of high fat diet for 8 weeks). For 4 weeks after injection, body weight and food intake have been measured. After 4 weeks from HD-Ad injection, blood was taken from tail vein of DIO mice fasted for 6 hours and the blood glucose level was measured. Immediately after that, plasma samples were collected by taking blood from heart and liver tissues were collected. RNA collected from liver and quantification of Acsl-1 mRNA expression were performed as well as the collection and quantification from cells in Example 4. Expression of liver Acsl-1 protein was analyzed by western blotting. In western blotting, Acsl-1 antibody (SANTA CRUZ BIOTECHNOLOGY: sc-49008) and HRP-Rabbit Anti-Goat IgG (H+L) conjugate (ZYMED Laboratories) were used. The above experiment was performed twice independently in each Acsl-1-shRNA.

In Experiment 1, HD-Ad-U6-Acsl1-12 (n=6) as an Acsl-1 knockdown group and HD-Ad-U6 (n=6) as a negative control group were injected and evaluated. In Experiment 2, HD-Ad-U6-Acsl1-16 (n=7) as an Acsl-1 knockdown group and HD-Ad-U6 (n=8) as a negative control group were injected and evaluated.

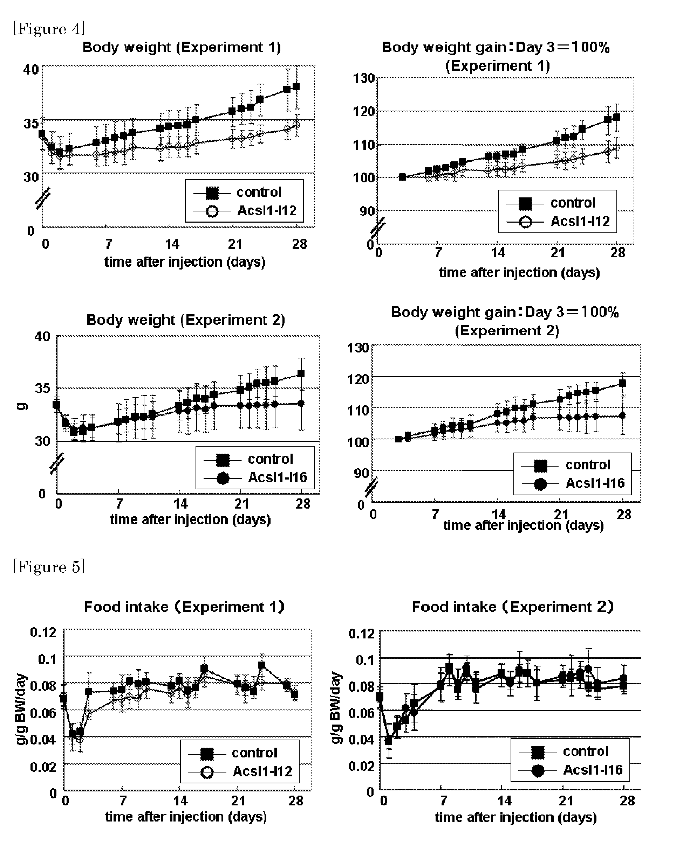

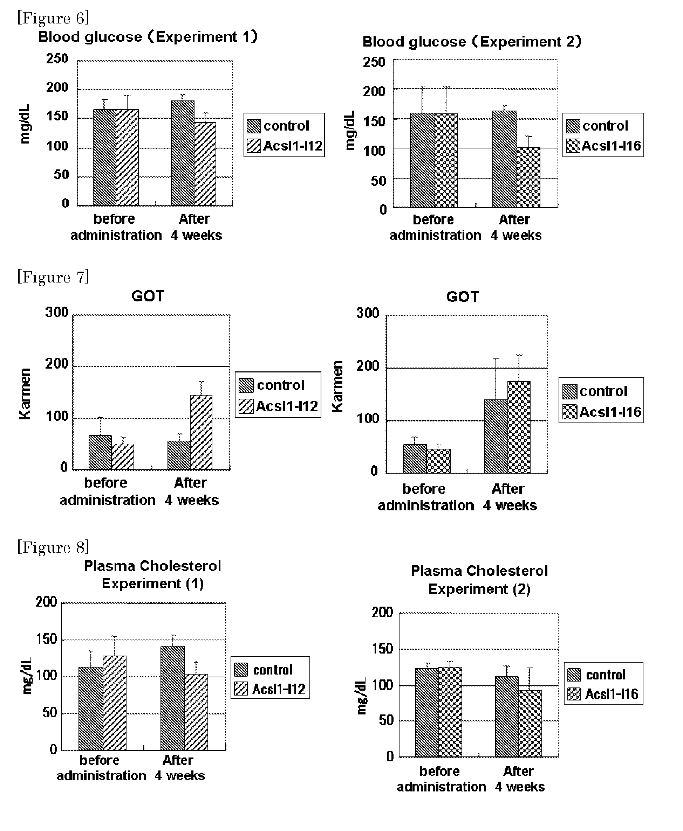

Result of analyzing liver Acsl-1 expression, mRNA level was decreased by 95% in HD-Ad-U6-Acsl1-12, and by 92% in HD-Ad-U6-Acsl1-16. The protein level has decreased up to the undetectable level in both groups injected Acsl-1 shRNA. Therefore, it was able to be confirmed to have succeeded in the reduction of liver Acsl-1 expression (Fig.3). Fig.4 and 5 shows the results of measuring body weight and food intake sequentially. As compared to control group, suppression of body weight gain was confirmed in Acsl-1 knockdown group, even though there is no difference in food intake (body weight: Experiment 1 p<0.005, Experiment 2: p<0.05, body weight gain: Experiment 1 p<0.005, Experiment 2: p<0.05). Following, Fig 6 shows the result of measuring blood glucose. As the data of administration of HD-Ad after 4 weeks shows, blood glucose level was decreased by Acsl-1 knockdown (Experiment 1 p<0.05, Experiment 2: p<0.001). As shown above, because both significant differences are seen, anti-obesity effect and improvement of hyperglycemia by suppressing Acsl-1 expression were confirmed.

### Example 7

### Glutamic c-oxaloacetic transaminase(GOT) measurement

To measure the effect of virus infection to hepatitis, plasma GOT level was measured. GOT was measured with the Transaminase CII TEST WAKO kit (Wako Pure Chemical Industries, Ltd.) according to the appended manual. In all the cases, Control, and Acsl-1 knockdown groups (Acsl-1 I12 and I16), slight increase in GOT level were occurred but the levels were thought to be not significant such as decreasing the body weight. Moreover, there were no differences among the GOT levels of each groups. Therefore, it can be confirmed that hepatitis does not arise by viral infection and Acsl-1 knockdown and the cause of weight decrease was not the hepatitis (Fig7).

### Example 8

### Measurement of the plasma insulin content

Plasma insulin levels were measured using Rebisu insulin mouse kit (Shibayagi) according to the appended manual. The measurement result is shown in Figure 8. As data of 4 weeks after HD-Ad infection shows, serum insulin level was decreased and insulin resistance was improved compared to control groups by repression of Acsl1 expression (Experiment 1 P<0.005, Experiment 2 P<0.001).

### Example 9

### Extraction of lipid component from liver

Folch method was used. 0.5 g of liver was homogenized and put into a microtube. 0.1 M KCL was added up to 1.4 ml at the volume and stirred a lot. And then it was transferred to 15 ml Falcon Tube. Next, after adding 6 ml of the mixture of CHCl₃ and MeOH, they were stirred for 1 hour at room temperature. 2 ml of sterilized water was added thereto and left for 5 minutes. They were centrifuged at 3000 rpm for 10 minutes and 720 µl of lower layer (organic phase) was recovered. The yellow clear lipid component was gained by evaporating the organic liquid for 20 minutes.

### Example 10

### Measurement of plasma and liver lipid component

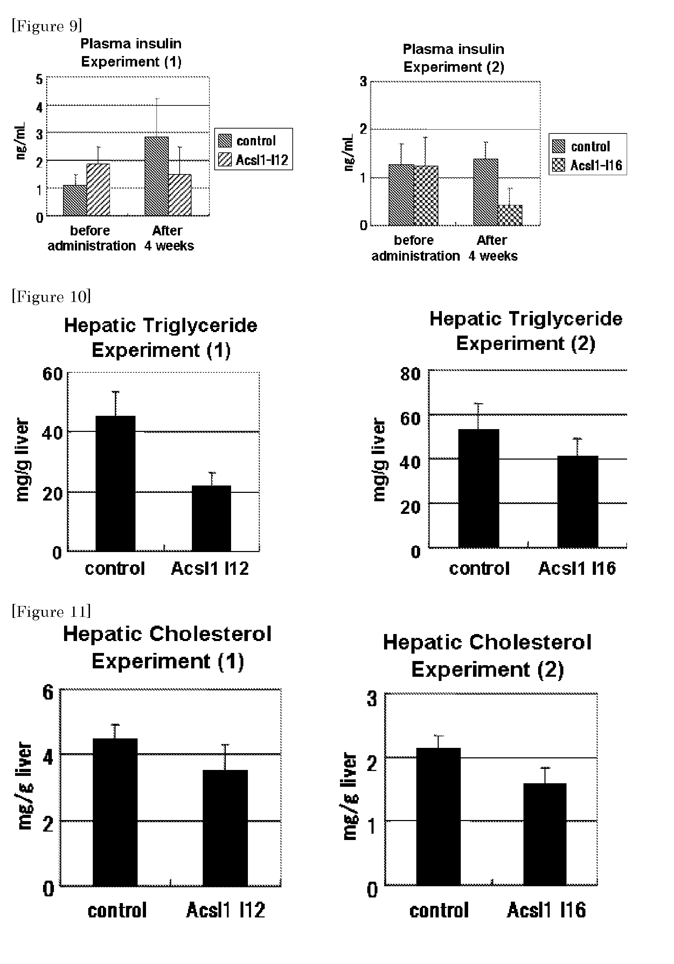

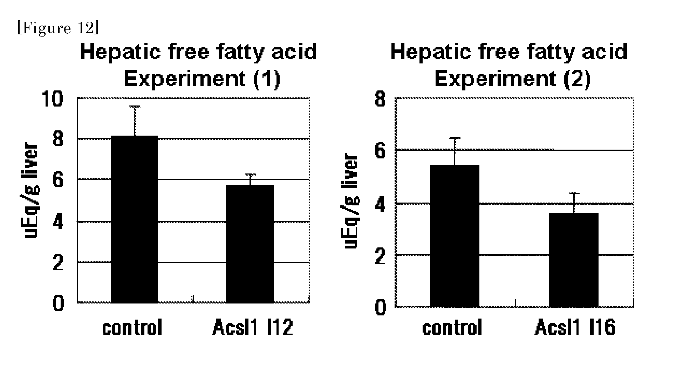

Triglyceride was measured using Triglyceride E-test WAKO (GPO·DAOS method: Wako Pure Chemical Industries, Ltd) according to the appended manual. Cholesterol was measured using Cholesterol E test WAKO (cholesterol oxidase·DAOS method: Wako Pure Chemical Industries, Ltd) according to the appended manual. Free fatty acid was measured using NEFA C-test WAKO (ACS·ACOD method: Wako Pure Chemical Industries, Ltd) according to the appended manual. The quantitative results of plasma total cholesterol are shown in Figure 9. The measurement results of liver triglyceride are shown in Figure 10. The measurement results of liver free cholesterol are shown in Figure 11. The measurement results of liver free fatty acid are shown in Figure 12. From the result of a measurement, the liver triglyceride (Experiment 1 P<0.001, Experiment 2 P<0.05), liver free fatty acid (Experiment 1 P<0.005, Experiment 2 P<0.005) and liver free cholesterol (Experiment 1 P<0.05, Experiment 2 P<0.001) were decreased compared to control groups by repressing the Acsl-1 expression and the improvement of fatty liver was confirmed. Moreover, decrease of plasma total cholesterol compared to control groups was confirmed (Experiment 1 P<0.005). Therefore, the improvement of metabolic syndrome was confirmed by repressing the Acsl-1 expression.

### Example 11

### The influence of repression of gene expression of Acsl-1 to lipid droplet accumulation in human hepatocyte cell line

The Hs_ACSL1_4_HP siRNA (SI00291228) and Hs_ACSL1_5_HP siRNA (S104244982) to the human Acsl-1 were purchased from QIAGEN. In order to use Diacyl glycerol acyl transferase (DGAT) that is the neutral lipid synthase as a positive control of this experiment, Hs_DGAT1_8_HP siRNA (SI03246754) to human DGAT1 and Hs_DGAT2_1_HP siRNA (SI00363087) to human DGAT2 were purchased from QIAGEN. An negative control (NC) siRNA was purchased from SAMCHULLY. Using the DMEM containing 10 % FCS (fatal calf serum), human hepatocyte cell line HepG2 was maintained at 5 % CO₂, 37 °C. Just before the transfection, HepG2 cells were seeded to 6 well plates. Reverse transfection of siRNA was performed with Lipofectamine RNAiMAX reagent (Invitrogen) at a final siRNA concentration of 50µM according to the appended manual. The used siRNA were Hs_ACSL1_4_HP siRNA, NC siRNA and mixture of Hs_DGAT1_8_HP and Hs_DGAT2_1_HP siRNA. Next day after the transfection, mixture of oleic acid and BSA (Bovine serum albumin) at 5:1 of mole ratio were prepared and added to the cell (Final concentration of oleic acid was 0.2 mM). 3 days after siRNA transfection (2 days after the addition of oleic acid), the lipid droplet accumulated in HepG2 cells were stained using triglyceride measuring reagent named AdipoRed (SANKO Chemical) according to the appended manual. After staining, HepG2 cells were detached from the culture dish and divided to two parts. One was used to measure the stained lipid droplet using FACS (BD) and another was used to collect RNA and measure the mRNA of Acsl-1, DGAT1 and DGAT2 by Real-time quantitative PCR. The used Real-time quantitative PCR primer's sequences to Acsl-1, DGAT1 and DGAT2 were shown in SEQ ID NO: 11 to 16 (RNA collection from cells and a quantitative method of mRNA are referred to Example 4).

From the result of analysis of Acsl-1, DGAT1 and DGAT2 in HepG2 cells, Hs_ACSL1_4_HP siRNA repressed the Acsl-1 mRNA level by 85 %, Hs_DGAT1_8_HP siRNA and Hs_DGAT2_1_HP siRNA repressed the DGAT1 and DGAT2 mRNA levels by 67 % and 50 % respectively. Under the condition which each gene is repressed, as a result that the lipid droplet accumulation was measured and compared to NC, the lipid droplet accumulation was repressed by knockdown of Acsl-1 by 27 % and by knockdown of DGAT1 and DGAT2 by 33 %. From the results, Acsl-1 inhibitors are expected to have repressing activity to lipid droplet accumulation to the same level as DGAT inhibitors. Moreover, because the same result was obtained using Hs_ACSL1_5_HP siRNA, this result was confirmed to be not an off-target effect.

### Example 12

### Design and synthesis of Acsl-1 siRNA

The continuous 19 mer sequences that were identical to both human Acsl-1 mRNA (NM-001995, SEQ ID NO: 9) and mouse Acsl-1 mRNA (NM-007981, SEQ ID NO: 10) were selected (Figure 13 to 17) for the following experiment. siRNAs that contain sense strand having 3' overhang with 2 bases of thymine (dTdT) and antisense strand having 3' overhang with 2 bases which are identical to corresponding Acsl-1 DNA sequence were synthesized. The synthesis of siRNA was ordered to SIGMA Aldrich JAPAN company. As a negative control, ALL Star Negative Control siRNA were purchased from QIAGEN.

### Example 13

### Confirmation of repressing activity of Acsl-1 siRNA

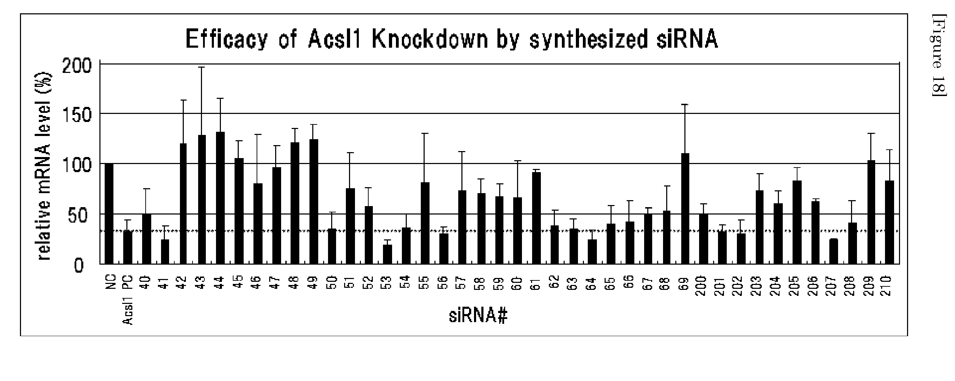

Using DMEM containing 10 % FCS at 5 % CO₂, 37 °C, HepG2 cells were maintained. Just before the transfection, HepG2 cells were seeded to 96 well plates. siRNA synthesized in Example 12, ALL STAR Negative control siRNA and Hs_ACSL1_4_HP siRNA used in Example 1 as a positive control were reverse transfected using Lipofectamine RNAiMAX reagent (Invitrogen) at a final siRNA concentration of 50µM according to the appended manual. 3 days after the transfection, the amount of Acsl-1 mRNA was measured using FastLane Cell SYBR Green Kit (QIAGEN) and Real-time quantitative PCR according to the appended manual and repression efficiency of each siRNA to gene expression of Acsl-1 were calculated. Partial results were shown in Figure 18. In this Figure, siRNAs having repression efficiency equal and more to Hs_ACSL1_4_HP siRNA (For example, siRNA produced based on the sequence of SEQ ID NO: 41, 53, 56, 64, 202 or 207 in Figure 13 to 17) was thought to have good repression efficiency of Acsl-1 expression and can be repress the lipid droplet accumulation. Furthermore, the pharmaceutical compositions comprising these siRNA with good repression efficiency of Acsl-1 expression are expected to use as an anti-obesity and anti-hyperglycemia drug. In the same way, as a result of the measurement of repressive effect of siRNAs with a wide variety of sequences, it turned out that siRNAs having sense strand indicated below have superior effect.

(sense sequence 5'→ 3', U: Uridine, A: Adenosine, G: Guanosine, C: Cytidine)
No.41, AUUUGUUUCACAAGUGGAA (SEQ ID NO: 17)
No.53, AGUGGAACUACAGGCAACC (SEQ ID NO: 18)
No.56, GGAACUACAGGCAACCCCA (SEQ ID NO: 19)
No.64, AGGCAACCCCAAAGGAGCA (SEQ ID NO: 20)
No.81, GCCUCUCGCCCAUAUGUUU (SEQ ID NO: 21)
No.98, GUAAUGCUGUGUCAUGGAG (SEQ ID NO: 22)
No.100, AAUGCUGUGUCAUGGAGCU (SEQ ID NO: 23)
No.101, AUGCUGUGUCAUGGAGCUA (SEQ ID NO: 24)
No.102, UGCUGUGUCAUGGAGCUAA (SEQ ID NO: 25)
No.111, CCAAGGAGAUAUCAGGCUG (SEQ ID NO: 26)
No.112, CAAGGAGAUAUCAGGCUGC (SEQ ID NO: 27)
No.117, GAAACAACAGCCUGUGGGA (SEQ ID NO: 28)
No.118, GAUGUGGAAGAAAUGAAUU (SEQ ID NO: 29)
No.119, AUGUGGAAGAAAUGAAUUA (SEQ ID NO: 30)
No.121, GAAAAGAUUGAAAAUAUCU (SEQ ID NO: 31)
No.125, AGGUGUUUGUCCACGGAGA (SEQ ID NO: 32)
No.126, GGUGUUUGUCCACGGAGAA (SEQ ID NO: 33)
No.137, GAACUAUUUCAGGUCGCAG (SEQ ID NO: 34)
No.138, AACUAUUUCAGGUCGCAGA (SEQ ID NO: 35)
No.139, ACUAUUUCAGGUCGCAGAU (SEQ ID NO: 36)
No.150, UCUCCAUGCAGUCAGUGGA (SEQ ID NO: 37)
No.153, AAGUUAAUUUGGGAAAUUA (SEQ ID NO: 38)
No.161, GGGGUUUUGAAUGUUUGCU (SEQ ID NO: 39)
No.163, GGUUUUGAAUGUUUGCUUU (SEQ ID NO: 40)
No.164, AGUGUUGGCUAUUUCUAUG (SEQ ID NO: 41)
No.165, GUGUUGGCUAUUUCUAUGU (SEQ ID NO: 42)
No.166, UGUUGGCUAUUUCUAUGUU (SEQ ID NO: 43)
No.178, CUAUGUUUUAUAAACCAAA (SEQ ID NO: 44)
No.190, AAAACAAUGAAGGAAACCA (SEQ ID NO: 45)
No.195, AAUGAAGGAAACCAAAAUA (SEQ ID NO: 46)
No.201, GGAAACCAAAAUAAAUAUU (SEQ ID NO: 47)
No.202, GAAACCAAAAUAAAUAUUU (SEQ ID NO: 48)
No.207, CAAAAUAAAUAUUUCUGCA (SEQ ID NO: 49)

The suppressive effect of each sequence is as below. The amount of Acsl-1 mRNA expression of NC siRNA was set as 100 and the amount of Acsl-1 mRNA when each siRNA was introduced was calculated and shown in Table.

**[Table 1]**

| siRNA# | Mean | standard deviation |
|---|---|---|
| NC | 100.0 | |
| Acsl1 PC | 31.5 | 11.7 |
| 41 | 22.8 | 15.1 |
| 53 | 17.9 | 6.4 |
| 56 | 30.2 | 6.7 |
| 64 | 24.1 | 9.9 |
| 81 | 30.2 | 12.4 |
| 98 | 22.6 | 16.6 |
| 100 | 20.7 | 17.0 |
| 101 | 21.1 | 16.0 |
| 102 | 26.2 | 18.5 |
| 111 | 26.0 | 9.7 |
| 112 | 21.3 | 10.4 |
| 117 | 23.4 | 13.5 |
| 118 | 18.9 | 10.1 |
| 119 | 16.3 | 8.7 |
| 121 | 26.5 | 9.2 |
| 125 | 20.3 | 10.3 |
| 126 | 31.4 | 6.1 |
| 136 | 24.0 | 14.4 |
| 137 | 26.9 | 15.6 |
| 138 | 24.4 | 12.9 |
| 139 | 19.7 | 3.3 |
| 150 | 26.2 | 11.6 |
| 153 | 29.0 | 7.6 |
| 161 | 24.4 | 7.5 |
| 163 | 15.2 | 2.2 |
| 164 | 26.9 | 7.4 |
| 165 | 20.1 | 7.5 |
| 166 | 29.4 | 14.9 |
| 178 | 29.3 | 12.7 |
| 190 | 22.5 | 7.7 |
| 195 | 27.0 | 11.5 |
| 201 | 31.4 | 7.5 |
| 202 | 29.8 | 13.8 |
| 207 | 23.3 | 2.0 |

## Claims

1. A pharmaceutical composition for preventing and/or treating obesity or type II diabetes **characterized by** comprising an Acsl-1 inhibitor as an active ingredient.

2. The composition of claim 1, wherein the inhibitor is a functional nucleic acid which suppresses Acsl-1 expression.

3. The composition of claim 2, wherein the functional nucleic acid is a siRNA.

4. The composition of claim 3, wherein the siRNA has the suppressive effect on lipid droplet accumulation.

5. The composition of claim 3, wherein the siRNA can suppress gene expression of Acsl-1 by 80% or more,

6. The composition of claim 5, wherein the siRNA is a siRNA having a sequence selected from the group consisting of SEQ ID NO: 17 to 49 as a RNA.

7. A method for treatment of obesity and /or type II diabetes **characterized by** administering an effective amount of an Acsl-1 inhibitor to a mammal.

8. The method for treatment of claim 7, wherein the inhibitor is a functional nucleic acid which suppresses Acsl-1 expression,

9. The method for treatment of claim 8, wherein the functional nucleic acid is a siRNA.

10. A screening method for a therapeutic agent of obesity or type II diabetes by using the inhibition of expression and /or action of Acsl-1 as an indicator.

11. A siRNA or derivative thereof wherein RNA has a sequence selected from the group consisting of SEQ ID NO: 17 to 49.

12. The siRNA of claim 11, further comprising a 3'-end overhang.


SEQUENCE LISTING

<110>  SHIONOGI&CO.,LTD

<120>  Antiobestic medical composition

<130>  09P00119WO

<160>  22

<170>  PatentIn version 3.1

<210>  1
<211>  3822
<212>  DNA
<213>  Homo sapiens

<400>  1
   tggctcagcc gcggcggtgg cgggggcgca accagcgggc cgaggcggcg gcgccagcgg      60
   cgccttaaat agcatccaga gccggcgcgg ggcagggagt gggctgcagt gacagccggc     120
   ggcggagcgg ccggtccacg gaggagaatt cagcttagag aactatcaac acaggacaat     180
   gcaagcccat gagctgttcc ggtattttcg aatgccagag ctggttgact tccgacagta     240
   cgtgcgtact cttccgacca acacgcttat gggcttcgga gcttttgcag cactcaccac     300
   cttctggtac gccacgagac ccaaacccct gaagccgcca tgcgacctct ccatgcagtc     360
   agtggaagtg gcgggtagtg gtggtgcacg aagatccgca ctacttgaca gcgacgagcc     420
   cttggtgtat ttctatgatg atgtcacaac attatacgaa ggtttccaga ggggaataca     480
   ggtgtcaaat aatggccctt gtttaggctc tcggaaacca gaccaaccct atgaatggct     540
   ttcatataaa caggttgcag aattgtcgga gtgcataggc tcagcactga tccagaaggg     600
   cttcaagact gccccagatc agttcattgg catctttgct caaaatagac ctgagtgggt     660
   gattattgaa caaggatgct ttgcttattc gatggtgatc gttccacttt atgataccct     720
   tggaaatgaa gccatcacgt acatagtcaa caaagctgaa ctctctctgg tttttgttga     780
   caagccagag aaggccaaac tcttattaga gggtgtagaa aataagttaa taccaggcct     840
   taaaatcata gttgtcatgg atgcctacgg cagtgaactg gtggaacgag gccagaggtg 900
   tggggtggaa gtcaccagca tgaaggcgat ggaggacctg ggaagagcca acagacggaa 960
   gcccaagcct ccagcacctg aagatcttgc agtaatttgt ttcacaagtg gaactacagg 1020
   caaccccaaa ggagcaatgg tcactcaccg aaacatagtg agcgattgtt cagcttttgt 1080
   gaaagcaaca gagaatacag tcaatccttg cccagatgat actttgatat ctttcttgcc 1140
   tctcgcccat atgtttgaga gagttgtaga gtgtgtaatg ctgtgtcatg gagctaaaat 1200
   cggatttttc caaggagata tcaggctgct catggatgac ctcaaggtgc ttcaacccac 1260
   tgtcttcccc gtggttccaa gactgctgaa ccggatgttt gaccgaattt tcggacaagc 1320
   aaacaccacg ctgaagcgat ggctcttgga ctttgcctcc aagaggaaag aagcagagct 1380
   tcgcagcggc atcatcagaa acaacagcct gtgggaccgg ctgatcttcc acaaagtaca 1440
   gtcgagcctg ggcggaagag tccggctgat ggtgacagga gccgccccgg tgtctgccac 1500
   tgtgctgacg ttcctcagag cagccctggg ctgtcagttt tatgaaggat acggacagac 1560
   agagtgcact gccgggtgct gcctgaccat gcctggagac tggaccgcag gccatgttgg 1620
   ggccccgatg ccgtgcaatt tgataaaact tgttgatgtg gaagaaatga attacatggc 1680
   tgccgagggc gagggcgagg tgtgtgtgaa agggccaaat gtatttcagg gctacttgaa 1740
   ggacccagcg aaaacagcag aagctttgga caaagacggc tggttacaca caggggacat 1800
   tggaaaatgg ttaccaaatg gcaccttgaa aattatcgac cggaaaaagc acatatttaa 1860
   gctggcacaa ggagaataca tagcccctga aaagattgaa aatatctaca tgcgaagtga 1920
   gcctgttgct caggtgtttg tccacggaga aagcctgcag gcatttctca ttgcaattgt 1980
   ggtaccagat gttgagacat tatgttcctg ggcccaaaag agaggatttg aagggtcgtt 2040
   tgaggaactg tgcagaaata aggatgtcaa aaaagctatc ctcgaagata tggtgagact 2100
   tgggaaggat tctggtctga aaccatttga acaggtcaaa ggcatcacat tgcaccctga 2160
   attatttict atcgacaatg gccttctgac tccaacaatg aaggcgaaaa ggccagagct 2220
   gcggaactat ttcaggtcgc agatagatga cctctattcc actatcaagg tttagtgtga 2280
   agaagaaagc tcagaggaaa tggcacagtt ccacaatctc ttctcctgct gatggccttc 2340
   atgttgttaa ttttgaatac agcaagtgta gggaaggaag cgttcgtgtt tgacttgtcc 2400
   attcggggtt cttctcatag gaatgctaga ggaaacagaa cactgcctta cagtcacctc 2460
   atgttgcaga ccatgtttat ggtaatacac actttccaaa atgagcctta aaaattgtaa 2520
   aggggatact ataaatgtgc taagttattt gagacttcct cagtttaaaa agtgggtttt 2580
   aaatcttctg tctccctgtt tttctaatca aggggttagg actttgctat ctctgagatg 2640
   tctgctactt gctgcaaatt ctgcagctgt ctgctgctct aaagagtaca gtgcactaga 2700
   gggaagtgtt ccctttaaaa ataagaacaa ctgtcctggc tggagaatct cacaagcgga 2760
   ccagagatct ttttaaatcc ctgctactgt cccttctcac aggcattcac agaacccttc 2820
   tgattcgtaa gggttacgaa actcatgttc ttctccagtc ccctgtggtt tctgttggag 2880
   cataaggttt ccagtaagcg ggagggcaga tccaactcag aaccatgcag ataaggagcc 2940
   tctggcaaat gggtgctcat cagaacgcgt ggattctctt tcatggcaga atgctcttgg 3000
   actcggttct ccaggcctga ttccccgact ccatcctttt tcaggggtta tttaaaaatc 3060
   tgccttagat tctatagtga agacaagcat ttcaagaaag agttacctgg atcagccatg 3120
   ctcagctgtg acgcctgaat aactgtctac tttatcttca ctgaaccact cactctgtgt 3180
   aaaggccaac agatttttaa tgtggttttc atatcaaaag atcatgttgg gattaacttg 3240
   cctttttccc caaaaaataa actctcaggc aagcatttct ttaaagctat taagggagta 3300
   tatacttgag tacttattga aatggacagt aataagcaaa tgttcttata atgctacctg 3360
   atttctatga aatgtgtttg acaagccaaa attctaggat gtagaaatct ggaaagttca 3420
   tttcctggga ttcacttctc cagggatttt ttaaagttaa tttgggaaat taacagcagt 3480
   tcactttatt gtgagtcttt gccacatttg actgaattga gctgtcattt gtacatttaa 3540
   agcagctgtt ttggggtctg tgagagtaca tgtattatat acaagcacaa cagggcttgc 3600
   actaaagaat tgtcattgta ataacactac ttggtagcct aacttcatat atgtattctt 3660
   aattgcacaa aaagtcaata atttgtcacc ttggggtttt gaatgtttgc tttaagtgtt 3720
   ggctatttct atgttttata aaccaaaaca aaatttccaa aaacaatgaa ggaaaccaaa 3780
   ataaatattt ctgcatttca ggtgaaaaaa aaaaaaaaaa aa 3822

<210> 2
<211> 698
<212> PRT
<213> Homo sapiens

<400> 2

Met Gln Ala His Glu Leu Phe Arg Tyr Phe Arg Met Pro Glu Leu Val
1               5                   10                  15

Asp Phe Arg Gln Tyr Val Arg Thr Leu Pro Thr Asn Thr Leu Met Gly
            20                  25                  30

Phe Gly Ala Phe Ala Ala Leu Thr Thr Phe Trp Tyr Ala Thr Arg Pro
        35                  40                  45

Lys Pro Leu Lys Pro Pro Cys Asp Leu Ser Met Gln Ser Val Glu Val
    50                  55                  60

Ala Gly Ser Gly Gly Ala Arg Arg Ser Ala Leu Leu Asp Ser Asp Glu
65                  70                  75                  80

Pro Leu Val Tyr Phe Tyr Asp Asp Val Thr Thr Leu Tyr Glu Gly Phe
                85                  90                  95

Gln Arg Gly Ile Gln Val Ser Asn Asn Gly Pro Cys Leu Gly Ser Arg
            100                 105                 110

Lys Pro Asp Gln Pro Tyr Glu Trp Leu Ser Tyr Lys Gln Val Ala Glu
        115                 120                 125

Leu Ser Glu Cys Ile Gly Ser Ala Leu Ile Gln Lys Gly Phe Lys Thr
    130                 135                 140

Ala Pro Asp Gln Phe Ile Gly Ile Phe Ala Gln Asn Arg Pro Glu Trp
145                 150                 155                 160

Val Ile Ile Glu Gln Gly Cys Phe Ala Tyr Ser Met Val Ile Val Pro
                165                 170                 175

Leu Tyr Asp Thr Leu Gly Asn Glu Ala Ile Thr Tyr Ile Val Asn Lys
            180                 185                 190

Ala Glu Leu Ser Leu Val Phe Val Asp Lys Pro Glu Lys Ala Lys Leu
        195                 200                 205

Leu Leu Glu Gly Val Glu Asn Lys Leu Ile Pro Gly Leu Lys Ile Ile
    210                 215                 220

Val Val Met Asp Ala Tyr Gly Ser Glu Leu Val Glu Arg Gly Gln Arg
225                 230                 235                 240

Cys Gly Val Glu Val Thr Ser Met Lys Ala Met Glu Asp Leu Gly Arg
                245                 250                 255

Ala Asn Arg Arg Lys Pro Lys Pro Pro Ala Pro Glu Asp Leu Ala Val
            260                 265                 270

Ile Cys Phe Thr Ser Gly Thr Thr Gly Asn Pro Lys Gly Ala Met Val
        275                 280                 285

Thr His Arg Asn Ile Val Ser Asp Cys Ser Ala Phe Val Lys Ala Thr
    290                 295                 300

Glu Asn Thr Val Asn Pro Cys Pro Asp Asp Thr Leu Ile Ser Phe Leu
305                 310                 315                 320

Pro Leu Ala His Met Phe Glu Arg Val Val Glu Cys Val Met Leu Cys
                325                 330                 335

His Gly Ala Lys Ile Gly Phe Phe Gln Gly Asp Ile Arg Leu Leu Met
            340                 345                 350

Asp Asp Leu Lys Val Leu Gln Pro Thr Val Phe Pro Val Val Pro Arg
        355                 360                 365

Leu Leu Asn Arg Met Phe Asp Arg Ile Phe Gly Gln Ala Asn Thr Thr
    370                 375                 380

Leu Lys Arg Trp Leu Leu Asp Phe Ala Ser Lys Arg Lys Glu Ala Glu
385                 390                 395                 400

Leu Arg Ser Gly Ile Ile Arg Asn Asn Ser Leu Trp Asp Arg Leu Ile
                405                 410                 415

Phe His Lys Val Gln Ser Ser Leu Gly Gly Arg Val Arg Leu Met Val
            420                 425                 430

Thr Gly Ala Ala Pro Val Ser Ala Thr Val Leu Thr Phe Leu Arg Ala
        435                 440                 445

Ala Leu Gly Cys Gln Phe Tyr Glu Gly Tyr Gly Gln Thr Glu Cys Thr
    450                 455                 460

Ala Gly Cys Cys Leu Thr Met Pro Gly Asp Trp Thr Ala Gly His Val
465                 470                 475                 480

Gly Ala Pro Met Pro Cys Asn Leu Ile Lys Leu Val Asp Val Glu Glu
                485                 490                 495

Met Asn Tyr Met Ala Ala Glu Gly Glu Gly Glu Val Cys Val Lys Gly
            500                 505                 510

Pro Asn Val Phe Gln Gly Tyr Leu Lys Asp Pro Ala Lys Thr Ala Glu
        515                 520                 525

Ala Leu Asp Lys Asp Gly Trp Leu His Thr Gly Asp Ile Gly Lys Trp
    530                 535                 540

Leu Pro Asn Gly Thr Leu Lys Ile Ile Asp Arg Lys Lys His Ile Phe
545                 550                 555                 560

Lys Leu Ala Gln Gly Glu Tyr Ile Ala Pro Glu Lys Ile Glu Asn Ile
                565                 570                 575

Tyr Met Arg Ser Glu Pro Val Ala Gln Val Phe Val His Gly Glu Ser
            580                 585                 590

Leu Gln Ala Phe Leu Ile Ala Ile Val Val Pro Asp Val Glu Thr Leu
        595                 600                 605

Cys Ser Trp Ala Gln Lys Arg Gly Phe Glu Gly Ser Phe Glu Glu Leu
    610                 615                 620

Cys Arg Asn Lys Asp Val Lys Lys Ala Ile Leu Glu Asp Met Val Arg
625                 630                 635                 640

Leu Gly Lys Asp Ser Gly Leu Lys Pro Phe Glu Gln Val Lys Gly Ile

645                 650                 655

Thr Leu His Pro Glu Leu Phe Ser Ile Asp Asn Gly Leu Leu Thr Pro
            660                 665                 670

Thr Met Lys Ala Lys Arg Pro Glu Leu Arg Asn Tyr Phe Arg Ser Gln
        675                 680                 685

Ile Asp Asp Leu Tyr Ser Thr Ile Lys Val
    690                 695

<210>  3
<211>  29
<212>  DNA
<213>  Artificial

<220>
<223>  primer

<400>  3
aaagcggccg cttgcctctc gcccatatg                                    29

<210>  4
<211>  27
<212>  DNA
<213>  Artificial

<220>
<223>  primer

<400>  4
aaactcgagt tagatcttga tggtggc                                      27

<210>  5
<211>  19
<212>  DNA
<213>  Artificial

<220>

<223>  primer

<400>  5
ggctgctgaa ccggatgtt                                                    19



<210>  6
<211>  21
<212>  DNA
<213>  Artificial

<220>
<223>  primer

<400>  6
gaagctccgc ctctttcctt t                                                 21



<210>  7
<211>  19
<212>  RNA
<213>  Artificial

<220>
<223>  oligonucleotide

<400>  7
ugccuugcaa uuauguaaa                                                    19



<210>  8
<211>  19
<212>  RNA
<213>  Artificial

<220>
<223>  oligonucleotide

<400>  8
auggcaccuu gaagauuau                                                    19



<210>  9
<211>  3822
<212>  RNA

<213>  Homo sapiens

<400>  9
uggcucagcc gcggcggugg cgggggcgca accagcgggc cgaggcggcg gcgccagcgg      60

cgccuuaaau agcauccaga gccggcgcgg ggcagggagu gggcugcagu gacagccggc     120

ggcggagcgg ccgguccacg gaggagaauu cagcuuagag aacuaucaac acaggacaau     180

gcaagcccau gagcuguucc gguauuuucg aaugccagag cugguugacu uccgacagua     240

cgugcguacu cuuccgacca acacgcuuau gggcuucgga gcuuuugcag cacucaccac     300

cuucugguac gccacgagac ccaaaccccu gaagccgcca ugcgaccucu ccaugcaguc     360

aguggaagug gcggguagug guggugcacg aagauccgca cuacuugaca gcgacgagcc     420

cuugguguau uucuaugaug augucacaac auuauacgaa gguuuccaga ggggaauaca     480

ggugucaaau aauggcccuu guuuaggcuc ucggaaacca gaccaacccu augaauggcu     540

uucauauaaa cagguugcag aauugucgga gugcauaggc ucagcacuga uccagaaggg     600

cuucaagacu gccccagauc aguucauugg caucuuugcu caaaauagac cugagugggu     660

gauuauugaa caaggaugcu uugcuuauuc gauggugauc guuccacuuu augauacccu     720

uggaaaugaa gccaucacgu acauagucaa caaagcugaa cucucucugg uuuuuguuga     780

caagccagag aaggccaaac ucuuauuaga ggguguagaa aauaaguuaa uaccaggccu     840

uaaaaucaua guugucaugg augccuacgg cagugaacug guggaacgag gccagaggug     900

ugggguggaa gucaccagca ugaaggcgau ggaggaccug ggaagagcca acagacggaa     960

gcccaagccu ccagcaccug aagaucuugc aguaauuugu uucacaagug gaacuacagg    1020

caaccccaaa ggagcaaugg ucacucaccg aaacauagug agcgauuguu cagcuuuugu    1080

gaaagcaaca gagaauacag ucaauccuug cccagaugau acuuugauau cuuucuugcc    1140

ucucgcccau auguuugaga gaguuguaga guguguaaug cugugucaug gagcuaaaau    1200

cggauuuuuc caaggagaua ucaggcugcu cauggaugac cucaaggugc uucaacccac    1260

ugucuucccc gugguuccaa gacugcugaa ccggauguuu gaccgaauuu ucggacaagc 1320

aaacaccacg cugaagcgau ggcucuugga cuuugccucc aagaggaaag aagcagagcu 1380

ucgcagcggc aucaucagaa acaacagccu gugggaccgg cugaucuucc acaaaguaca 1440

gucgagccug ggcggaagag uccggcugau ggugacagga gccgccccgg ugucugccac 1500

ugugcugacg uuccucagag cagcccuggg cugucaguuu uaugaaggau acggacagac 1560

agagugcacu gccgggugcu gccugaccau gccuggagac uggaccgcag gccauguugg 1620

ggccccgaug ccgugcaauu ugauaaaacu uguugaugug gaagaaauga auuacauggc 1680

ugccgagggc gagggcgagg ugugugugaa agggccaaau guauuucagg gcuacuugaa 1740

ggacccagcg aaaacagcag aagcuuugga caaagacggc ugguuacaca caggggacau 1800

uggaaaaugg uuaccaaaug gcaccuugaa aauuaucgac cggaaaaagc acauauuuaa 1860

gcuggcacaa ggagaauaca uagccccuga aaagauugaa aauaucuaca ugcgaaguga 1920

gccuguugcu cagguguuug uccacggaga aagccugcag gcauuucuca uugcaauugu 1980

gguaccagau guugagacau uauguuccug ggcccaaaag agaggauuug aagggucguu 2040

ugaggaacug ugcagaaaua aggaugucaa aaaagcuauc cucgaagaua uggugagacu 2100

ugggaaggau ucuggucuga aaccauuuga acaggucaaa ggcaucacau ugcacccuga 2160

auuauuuucu aucgacaaug gccuucugac uccaacaaug aaggcgaaaa ggccagagcu 2220

gcggaacuau uucaggucgc agauagauga ccucuauucc acuaucaagg uuuaguguga 2280

agaagaaagc ucagaggaaa uggcacaguu ccacaaucuc uucuccugcu gauggccuuc 2340

auguuguuaa uuuugaauac agcaagugua gggaaggaag cguucguguu ugacuugucc 2400

auucgggguu cuucucauag gaaugcuaga ggaaacagaa cacugccuua cagucaccuc 2460

auguugcaga ccauguuuau gguaauacac acuuuccaaa augagccuua aaaauuguaa 2520

aggggauacu auaaaugugc uaaguuauuu gagacuuccu caguuuaaaa agugguuuuu 2580

aaaucuucug ucucccuguu uuucuaauca agggguuagg acuuugcuau cucugagaug 2640

ucugcuacuu gcugcaaauu cugcagcugu cugcugcucu aaagaguaca gugcacuaga 2700

gggaaguguu cccuuuaaaa auaagaacaa cuguccuggc uggagaaucu cacaagcgga 2760

ccagagaucu uuuuaaaucc cugcuacugu cccuucucac aggcauucac agaacccuuc 2820

ugauucguaa ggguuacgaa acucauguuc uucuccaguc cccugugguu ucuguuggag 2880

cauaagguuu ccaguaagcg ggagggcaga uccaacucag aaccaugcag auaaggagcc 2940

ucuggcaaau gggugcucau cagaacgcgu ggauucucuu ucauggcaga augcucuugg 3000

acucgguucu ccaggccuga uuccccgacu ccauccuuuu ucagggguua uuuaaaaauc 3060

ugccuuagau ucuauaguga agacaagcau uucaagaaag aguuaccugg aucagccaug 3120

cucagcugug acgccugaau aacugucuac uuuaucuuca cugaaccacu cacucugugu 3180

aaaggccaac agauuuuuaa ugugguuuuc auaucaaaag aucauguugg gauuaacuug 3240

ccuuuuuccc caaaaaauaa acucucaggc aagcauuucu uuaaagcuau uaagggagua 3300

uauacuugag uacuuauuga aauggacagu aauaagcaaa uguucuuaua augcuaccug 3360

auuucuauga aauguguuug acaagccaaa auucuaggau guagaaaucu ggaaaguuca 3420

uuuccuggga uucacuucuc cagggauuuu uuaaaguuaa uuugggaaau uaacagcagu 3480

ucacuuuauu gugagucuuu gccacauuug acugaauuga gcugucauuu guacauuuaa 3540

agcagcuguu uuggggucug ugagaguaca uguauuauau acaagcacaa cagggcuugc 3600

acuaaagaau ugucauugua auaacacuac uugguagccu aacuucauau auguauucuu 3660

aauugcacaa aaagucaaua auuugucacc uugggguuuu gaauguuugc uuuaaguguu 3720

ggcuauuucu auguuuuaua aaccaaaaca aaauuuccaa aaacaaugaa ggaaaccaaa 3780

auaaauauuu cugcauuuca ggugaaaaaa aaaaaaaaaa aa 3822

<210> 10
<211> 3891
<212> RNA

<213>  mouse

<400>  10
gggcggggcg gcguggcacg gcacgcgcac uaccagccgc ccggcccugc ugcugcugcu      60

gcaacgccgg uggcgcagcc aagagagcag cugcggccgc gacuccuuaa auagcaucgc     120

aacccggcgc ggggcaguuc guggauugca gugacagccg ccuguggagc agcccgggca     180

gcggaggaga auucugcaua gagaaccacc aacccagaac cauggaaguc caugaauugu     240

uccgguauuu ucgaaugcca gagcugauug acauucggca guacgugcgc acccuuccaa     300

ccaacacccu caugggguuu ggggcuuuug cagcgcucac caccuucugg uaugccacca     360

ggccuaaggc ccugaagcca ccaugugacc ucuccaugca gucaguggaa auagcgggua     420

ccacugaugg uauucgaaga ucagcagucc uugaagauga caagcucuug guguacuacu     480

acgacgaugu cagaaccaug uacgauggcu uccagagggg gauucaggug ucaaauaaug     540

guccuuguuu agguucucgg aagccaaacc agcccuauga guggauuucc uacaaagagg     600

uggcagaacu ggcugagugc auaggcuccg ggcugaucca gaagggguuc aagccuugcu     660

ccgagcaguu caucggccuc uucucucaaa acagacccga gugggugauc gucgagcaag     720

gaugcuucuc uuacucaaug gugguguccc cgcucuauga cacccuugga gcugacgcca     780

ucaccuacau agugaacaaa gcugaacucu cugugauuuu ugcugacaag ccagaaaaag     840

ccaaacucuu auuagaaggu guagaaaaca aguuaacacc augccuuaaa aucauaguca     900

ucauggacuc cuacggcagu gaucuggugg aacgaggcaa gaaguguggg guggaaauca     960

ucagccucaa agcucuggag gaccuuggaa gagugaacag agugaagccc aagccuccag    1020

aacccgaaga ucuugcgaua auuuguuuca caaguggaac uacaggcaac cccaaaggag    1080

caaugaucac ucaccaaaac auuauaaacg acugcucagg uuuuauaaaa gcaacagaga    1140

gugcauucau cgcuuccaca gaugaugugc ugauaucuuu cuugccucuc gcccauaugu    1200

uugagaccgu uguagagugu guaaugcugu gucauggagc uaagauagga uuuuuccaag    1260

gagauaucag gcugcuuaug gacgaccuca aggugcuuca gcccaccauc uucccugugg 1320

uucccaggcu gcugaaccgg auguucgaca gaauuuuugg acaagcaaac acuuccuuga 1380

agcgauggcu guuggacuuu gccuccaaaa ggaaagaggc ggagcuucgc aguggcaucg 1440

ucagaaacaa cagccugugg gauaaacuca ucuuccacaa gauacagucg agccugggug 1500

ggaaaguccg gcugaugauc acaggagcag ccccgguguc ugccacagug cugacguuuc 1560

ugaggacagc gcucggcugc caguucuaug aaggcuacgg acagaccgag ugcacugcug 1620

guugcugccu gagcuugccc ggagacugga cggcaggcca uguuggagcc cccaugccuu 1680

gcaauuaugu aaagcuugug gauguggaag aaaugaauua ccuggcaucc aagggcgagg 1740

gugaggugug ugugaaaggg gcaaaugugu ucaaaggcua cuugaaagac ccagcaagaa 1800

cagcugaagc ccuggauaaa gauggcuggu uacacacggg ggacauugga aaauggcugc 1860

caaauggcac cuugaagauu aucgacagga aaaagcacau auuuaaacua gcccaaggag 1920

aguacauagc accagaaaag auugaaaaua ucuaccugcg gagugaagcc guggcccagg 1980

uguuugucca cggagaaagc uugcaggccu uucucauagc aguuguggua cccgacguug 2040

agagccuacc guccugggca cagaagagag gcuuacaagg guccuucgaa gaacugugca 2100

ggaacaagga uaucaauaaa gcuauccugg acgacuuguu gaaacuuggg aaggaagccg 2160

gucugaagcc auuugaacag gucaaaggca uugcugugca cccggaauua uuuucuauug 2220

acaacggccu ucugacucca acacugaagg cgaagaggcc agagcuacgg aacuauuuca 2280

ggucgcagau agaugaacug uacgccacca ucaagaucua acgugaggaa ggauacuuag 2340

aagaaauggc gcaucuccac aauccuccuc guaccaaugg ccuucgaguu gguaacuuug 2400

ccugcagcga gugugggaaa ggaaaugccc ugccgccgga cuuguccacg gggucuuacc 2460

auagggauag ucgagggcac ggaacacugc cuuacuuaca gucaccugug uugcagccca 2520

ugaucccggg gacacacaau uuccaaaacg agccuuaaac auuguaaagg ggaacccaua 2580

aaagugcuaa guuauuuaag acuucuucaa ccaauaaggu ggaugcuaca aguucugucu 2640

ccuguuuuuc uaacugaggg guuaggacuu auucuuucug auaugucugc ugcuugcugc 2700

gcguuuugca gcugucugcu gcucugaaga gcaccguaca cuggaggaaa gcugucccuu 2760

uaagaacaac uguccaggcu gaagaaaguc acaguggacc agagguuuuc cuuuugaacc 2820

ccuccucccc cuugccccuu ucccucacca ccucacauac aguacacuca caugaccuuu 2880

cugguuugua aggguuccac acggucccug uuugcgccug cuggaacaug agguuuucag 2940

uaaacuaaag agcagacccu cuucagaaca uguggguguc cagucucaga aagaaaaaaa 3000

aaauggcggc ucacucaaac guucucagac ucccucccaa aucuggucuc cugccucccu 3060

cccccucguc ccagggguua uaagaaaucc ugccuuagac ucugcaauga agaugguauu 3120

ucaaagaacg cccuggagcg gccaugcuca acugugacau cuccaccgcu cuccaccugc 3180

ucccucgugg aagcagccca uguugguaau ggccacuggg cucccucgug gaagcagccc 3240

auguugguaa cggccacugg gcucccucgu gguuuuccug gcaaaugaua augcggugau 3300

uaccuugcuu uuuuuccccu cccaguuaaa cucaggcaaa gcaugucuuc aaaacuaugu 3360

agggagcuga uauacauggg uacuuauuaa aauggacagu aacaaguaaa uauccuuauu 3420

auacaacuac cugauuuaug ugaaaugcau uugacaagcc aaaaaucugg gaugaagaaa 3480

ucugagggga auaaaaucau acccugggau ucacuuccca agguauauau auuuuuuaag 3540

uuaauuuggg aaauuaacag cauuucacuu uacugcaagu cuuugccaca uccgaccuau 3600

ccaagcuguu guuuauacau uugaagccac uguuguuggc ugugugacag ugcguguguu 3660

auugaagcac aacuggguuu gcacuaaaga aucaucauug uaauaacacu auuugguagc 3720

cuaacuucau acucguauuc uuaauugcac aggaagucgc uaauuuguca caacgggguu 3780

uugaauguuu gcuuucagug uuggcuauuu cuauguuuua uaaaccaaaa cagaauuucc 3840

aaaaacaaug aaggaaacca aaauaaauau uucugcauuu ggagugagug g 3891

<210> 11

<211>  23
<212>  DNA
<213>  Artificial

<220>
<223>  primer for PCR

<400>  11
aagaacaact gtcctggctg gag                                              23


<210>  12
<211>  22
<212>  DNA
<213>  Artificial

<220>
<223>  primer for PCR

<400>  12
agggttctgt gaatgcctgt ga                                               22


<210>  13
<211>  20
<212>  DNA
<213>  Artificial

<220>
<223>  primer for PCR

<400>  13
agcaagtgga tggccaggac                                                  20


<210>  14
<211>  19
<212>  DNA
<213>  Artificial

<220>
<223>  primer for PCR

<400>  14
gaggcggaac attcgcaga                                                   19

<210>  15
<211>  20
<212>  DNA
<213>  Artificial

<220>
<223>  primer for PCR

<400>  15
cctggcaaga atgcagtcac                                                    20



<210>  16
<211>  23
<212>  DNA
<213>  Artificial

<220>
<223>  primer for PCR

<400>  16
tcctcgaaga tcacctgctt gta                                                23



<210>  17
<211>  19
<212>  RNA
<213>  Artificial

<220>
<223>  sense strand of siRNA

<400>  17
auuuguuuca caaguggaa                                                     19



<210>  18
<211>  19
<212>  RNA
<213>  Artificial

<220>
<223>  sense strand of siRNA

<400>  18
aguggaacua caggcaacc                                                    19

<210>  19
<211>  19
<212>  RNA
<213>  Artificial

<220>
<223>  sense strand of siRNA

<400>  19
ggaacuacag gcaacccca                                                    19

<210>  20
<211>  19
<212>  RNA
<213>  Artificial

<220>
<223>  sense strand of siRNA

<400>  20
aggcaacccc aaaggagca                                                    19

<210>  21
<211>  19
<212>  RNA
<213>  Artificial

<220>
<223>  sense strand of siRNA

<400>  21
gccucucgcc cauauguuu                                                    19

<210>  22
<211>  19
<212>  RNA
<213>  Artificial

<220>
<223>  sense strand of siRNA

<400>  22
guaaugcugu gucauggag                                                    19


<210>  23
<211>  19
<212>  RNA
<213>  Artificial

<220>
<223>  sense strand of siRNA

<400>  23
guaaugcugu gucauggag                                                    19


<210>  24
<211>  19
<212>  RNA
<213>  Artificial

<220>
<223>  sense strand of siRNA

<400>  24
aaugcugugu cauggagcu                                                    19


<210>  25
<211>  19
<212>  RNA
<213>  Artificial

<220>
<223>  sense strand of siRNA

<400>  25
augcuguguc auggagcua                                                    19


<210>  26

<211>  19
<212>  RNA
<213>  artificial

<220>
<223>  sense strand of siRNA

<400>  26
ugcuguguca uggagcuaa                                                    19


<210>  27
<211>  19
<212>  RNA
<213>  Artificial

<220>
<223>  sense strand of siRNA

<400>  27
ccaaggagau aucaggcug                                                    19


<210>  28
<211>  19
<212>  RNA
<213>  Artificial

<220>
<223>  sense strand of siRNA

<400>  28
caaggagaua ucaggcugc                                                    19


<210>  29
<211>  19
<212>  RNA
<213>  Artificial

<220>
<223>  sense strand of siRNA

<400>  29
gaaacaacag ccuguggga                                                    19

<210>  30
<211>  19
<212>  RNA
<213>  Artificial

<220>
<223>  sense strand of siRNA

<400>  30
gauguggaag aaaugaauu                                                    19

<210>  31
<211>  19
<212>  RNA
<213>  Artificial

<220>
<223>  sense strand of siRNA

<400>  31
auguggaaga aaugaauua                                                    19

<210>  32
<211>  19
<212>  RNA
<213>  Artificial

<220>
<223>  sense strand of siRNA

<400>  32
gaaaagauug aaaauaucu                                                    19

<210>  33
<211>  19
<212>  RNA
<213>  Artificial

<220>
<223>  sense strand of siRNA

<400>  33
agguguuugu ccacggaga                                                    19

<210>  34
<211>  19
<212>  RNA
<213>  Artificial

<220>
<223>  sense strand of siRNA

<400>  34
gguguuuguc cacggagaa                                                    19

<210>  35
<211>  19
<212>  RNA
<213>  Artificial

<220>
<223>  sense strand of siRNA

<400>  35
gaacuauuuc aggucgcag                                                    19

<210>  36
<211>  19
<212>  RNA
<213>  Artificial

<220>
<223>  sense strand of siRNA

<400>  36
aacuauuuca ggucgcaga                                                    19

<210>  37
<211>  19
<212>  RNA
<213>  Artificial

<220>
<223>  sense strand of siRNA

<400>  37
acuauuucag gucgcagau                                                    19

<210>  38
<211>  19
<212>  RNA
<213>  Artificial

<220>
<223>  sense strand of siRNA

<400>  38
aaguuaauuu gggaaauua                                                    19

<210>  39
<211>  19
<212>  RNA
<213>  Artificial

<220>
<223>  sense strand of siRNA

<400>  39
   gggguuuuga auguuugcu                                                    19

<210>  40
<211>  19
<212>  RNA
<213>  Artificial

<220>
<223>  sense strand of siRNA

<400>  40
gguuuugaau guuugcuuu                                                    19

<210>  41

<211>  19
<212>  RNA
<213>  Artificial

<220>
<223>  sense strand of siRNA

<400>  41
aguguuggcu auuucuaug                                                    19


<210>  42
<211>  19
<212>  RNA
<213>  Artificial

<220>
<223>  sense strand of siRNA

<400>  42
guguuggcua uuucuaugu                                                    19


<210>  43
<211>  19
<212>  RNA
<213>  Artificial

<220>
<223>  sense strand of siRNA

<400>  43
uguuggcuau uucuauguu                                                    19


<210>  44
<211>  19
<212>  RNA
<213>  Artificial

<220>
<223>  sense strand of siRNA

<400>  44
cuauguuuua uaaaccaaa                                                    19

<210>  45
<211>  19
<212>  RNA
<213>  Artificial

<220>
<223>  sense strand of siRNA

<400>  45
aaaacaauga aggaaacca                                                    19



<210>  46
<211>  19
<212>  RNA
<213>  Artificial

<220>
<223>  sense strand of siRNA

<400>  46
aaugaaggaa accaaaaua                                                    19



<210>  47
<211>  19
<212>  RNA
<213>  Artificial

<220>
<223>  sense  strand of siRNA

<400>  47
ggaaaccaaa auaaauauu                                                    19



<210>  48
<211>  19
<212>  RNA
<213>  Artificial

<220>
<223>  sense strand of siRNA

<400> 48
gaaaccaaaa uaaauauuu 19

<210> 49
<211> 19
<212> RNA
<213> Artificial

<220>
<223> sense strand of siRNA

<400> 49
caaaauaaau auuucugca 19